(19) Europäisches Patentamt
European Patent Office
Office européen des brevets

(11) Numéro de publication: **0 308 345 B1**

(12) # FASCICULE DE BREVET EUROPEEN

(45) Date de publication de fascicule du brevet: **28.04.93**

(51) Int. Cl.[5]: **C07J 21/00**, C07J 41/00, C07J 31/00, A61K 31/58, C07J 43/00, //C07J51/00

(21) Numéro de dépôt: **88402344.1**

(22) Date de dépôt: **16.09.88**

Le dossier contient des informations techniques présentées postérieurement au dépôt de la demande et ne figurant pas dans le présent fascicule.

(54) **Nouveaux stéroides comportant un cycle spirannique à 3, 4 ou 6 chaînons en position 17, leur procédé et des intermédiaires de préparation, leur application comme médicaments et les compositions pharmaceutiques les renfermant.**

(30) Priorité: **18.09.87 FR 8712937**

(43) Date de publication de la demande: **22.03.89 Bulletin 89/12**

(45) Mention de la délivrance du brevet: **28.04.93 Bulletin 93/17**

(84) Etats contractants désignés: **AT BE CH DE ES FR GB GR IT LI LU NL SE**

(56) Documents cités:
**EP-A- 262 188**
**EP-A- 0 097 572**
**EP-A- 0 116 974**
**EP-A- 0 156 284**

(73) Titulaire: **ROUSSEL-UCLAF**
**35, Boulevard des Invalides**
**F-75007 Paris(FR)**

(72) Inventeur: **Nedelec, Lucien**
**45, Boulevard de l'Ouest**
**F-93340 Le Raincy(FR)**
Inventeur: **Claussner, André**
**62, Rue Marc Vieville**
**F-93250 Villemomble(FR)**
Inventeur: **Philibert, Daniel**
**16, rue Chevalier**
**F-94210 La Varenne Saint-Hilaire(FR)**
Inventeur: **Moguilewsky, Martine**
**37, rue Lamartine**
**F-75009 Paris(FR)**

(74) Mandataire: **Bourgouin, André et al**
**Département des Brevets ROUSSEL UCLAF**
**111, route de Noisy B.P. no 9**
**F-93230 Romainville (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.5x/3.0.1)

## Description

La présente invention concerne de nouveaux stéroïdes comportant un cycle spirannique à 3,4 ou 6 chaînons en position 17, leur procédé et des intermédiaires de préparation, leur application comme médicaments et les compositions pharmaceutiques les renfermant.

Des produits comportant un cycle spirannique en position 17 mais ne comportant pas de radical arylique en position 11 sont décrits dans le brevet européen EP 0.156.284.

Des produits comportant un radical arylique en position 11 sont décrits dans les brevets européens EP 0.097.572 et 0.116.974. Dans ce dernier brevet sont décrits notamment des produits comportant un cycle lactonique en positon 17. Les produits de la présente demande présentent, par rapport à ces produits, une affinité plus grande pour des récepteurs hormonaux.

L'invention a pour objet les produits de formule générale (I) :

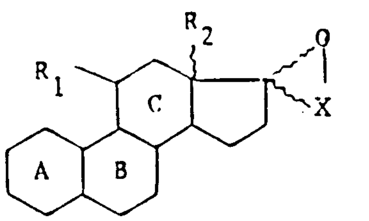

(I)

dans laquelle $R_1$ représente un radical aryle ou aralkyle carbocyclique ou hétérocyclique éventuellement substitué, $R_2$ en position alpha ou béta, représente un radical hydrocarboné renfermant de 1 à 18 atomes de carbone, le trait ondulé du spiro éther indique que l'atome d'oxygène peut se trouver en position alpha ou béta, X représente un radical $(CH_2)_n$- dans lequel n représente un entier égal à 1,2 ou 4, ou X représente un radical $-CH=CH-CH_2-CH_2-$ les cycles A et B ayant l'une des structures suivantes :

a) - soit A et B représentent le groupement :

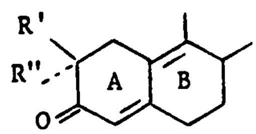

dans lequel R′ et R″ identiques ou différents représentent un atome d'hydrogène, ou un radical alkyle ayant de 1 à 4 atomes de carbone,

b) - soit A, B représentent le groupement :

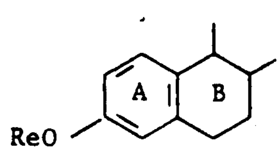

dans lequel Re représente un atome d'hydrogène, un radical alkyle ayant de 1 à 6 atomes de carbone éventuellement substitué ou un radical acyle ;

c) - soit A, B et C représentent le groupement :

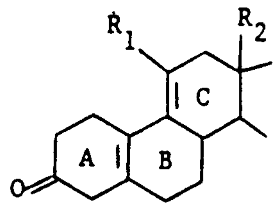

d) - soit A et B représentent le groupement :

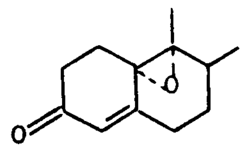

e) - soit A et B représentent le groupement :

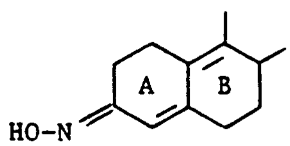

ainsi que leurs sels et lorsque X représente $-CH_2-$, de préférence $R'$ et $R''$ représentent chacun un atome d'hydrogène.

Lorsque $R_1$ représente un radical aryle ou aralkyle carbocyclique éventuellement substitué, il s'agit de préférence du radical phényle ou benzyle. Ces radicaux aromatiques peuvent être substitués en ortho, méta ou para par un ou plusieurs radicaux alkyles renfermant de préférence de 1 à 8 atomes de carbone ; par un ou plusieurs radicaux alkoxy ayant préférentiellement de 1 à 8 atomes de carbone tels que les radicaux méthoxy, éthoxy, propyloxy, isopropyloxy, butyloxy, isobutyloxy, tert-butyloxy, alkényloxy tel que vinyloxy ou allyloxy tous ces radicaux étant éventuellement substitués ; par un ou plusieurs atomes d'halogène, tels que fluor, chlore, brome, iode, de préférence chlore ou fluor ; par un ou plusieurs radicaux choisis parmi les radicaux hydroxyle, trifluorométhyle, acyle ayant de 1 à 6 atomes de carbone tel que acétyle, propionyle, carboxy éventuellement estérifié tel que méthoxycarbonyle, éthoxycarbonyle, propoxycarbonyle, alkylthio ayant de 1 à 8 atomes de carbone tel que méthylthio, éthylthio éventuellement oxydé sous forme de sulfoxyde ou de sulfone ; par un ou plusieurs radicaux amino ou amino mono ou disubstitués par les radicaux alkyles comportant de 1 à 8 atomes de carbone, eux-mêmes éventuellement substitués tels que les radicaux méthylamino, diméthylamino et bis (chloroéthyl) amino, les radicaux amino ou amino mono ou disubstitués étant éventuellement oxydés en N-oxyde, les radicaux amino incorporés dans un hétérocycle comportant éventuellement un hétéroatome choisi dans le groupe formé par l'oxygène, l'azote et le soufre tel que les radicaux morpholino ou pipéridinyle ; bien entendu les radicaux aryle ou aralkyle peuvent être substitués par une combinaison de ces différents radicaux tel que par exemple 2-méthylthioéthoxy, 3-fluoro, 4-diméthylamino ; $R_1$ peut également représenter un radical aryle hétérocyclique éventuellement substitué par les différents radicaux envisagés ci-dessus. On peut citer les radicaux thiényle, furyle, isothiényle, isofuryle, thiazolyle, isothiazolyle, oxazolyle, isoxazolyle, thiadiazolyle, pyridinyle ou pipéridinyle et les hétérocycles connus de l'homme de métier.

Comme substituant sur le noyau arylique, on peut également envisager un radical amino (substitué) alkyle tel que le radical diméthylamino méthyle, diméthylamino éthyle, méthyl (diméthyl aminoéthyl) amino ; un radical amino (substitué) alkyloxy tel que le radical diméthylamino éthyloxy.

On peut également citer les radicaux comportant un atome de silicium tel que le radical triméthylsilyl phényle.

Les radicaux précédemment cités comportant un atome d'azote ou de soufre peuvent être oxydés.

De manière générale, on préfère les produits dans lesquels le substituant $R_1$ comporte un hétéroatome de préférence l'azote ou le soufre.

Parmi les différents radicaux $R_1$, on peut citer les radicaux suivants :

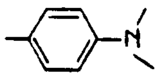 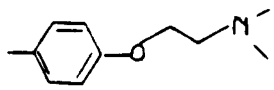 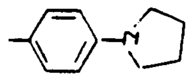 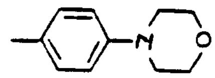

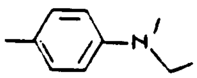 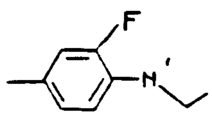 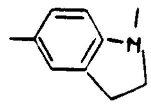 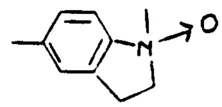

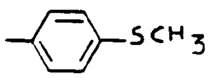 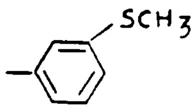 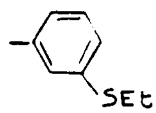 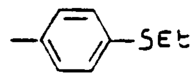 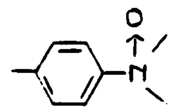

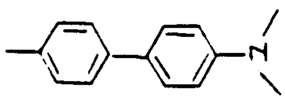 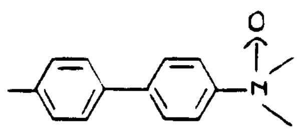 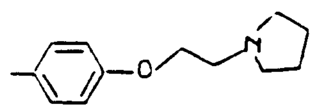

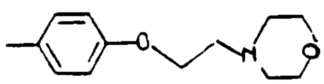 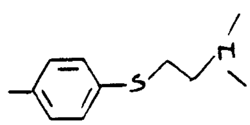 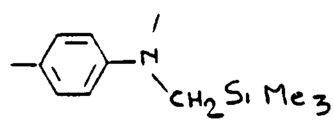

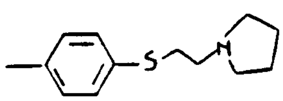 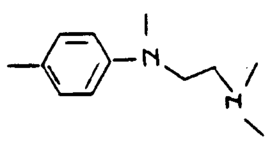 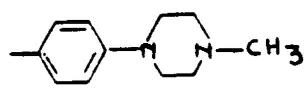

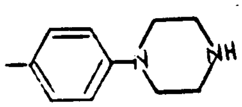

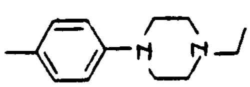 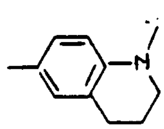 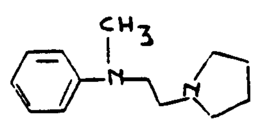 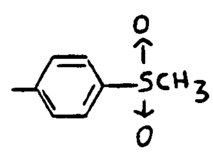

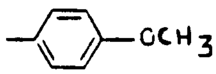
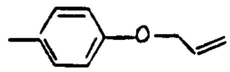
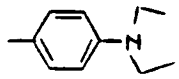
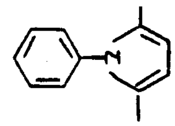

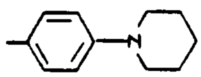
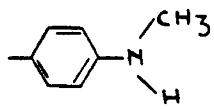
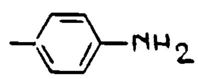
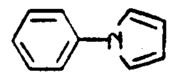
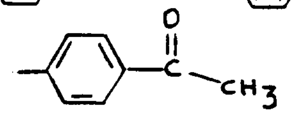

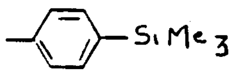
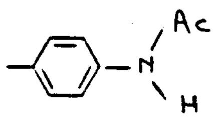
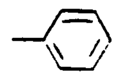
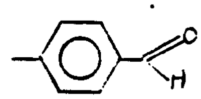

Le radical $R_2$ représente de préférence un radical alkyle saturé, linéaire ou ramifié, renfermant de 1 à 4 atomes de carbone, par exemple, un radical méthyle, éthyle, propyle ou butyle.

Préférentiellement $R_2$ représente un radical méthyle ou éthyle.

Plus préférentiellement $R_2$ représente un radical méthyle.

Le radical $R_2$ peut être en position alpha ou béta. On préfère les produits dans lesquels $R_2$ est en position béta. Dans ce dernier cas, l'atome d'oxygène de la fonction spiro éther est en position 17béta.

Lorsque $R_1$ comporte une fonction carboxy, celle-ci peut être salifiée. Parmi les sels possibles, on peut citer, par exemple, les sels de sodium, de potassium, de lithium, de calcium, de magnésium ou d'ammonium. On peut citer, parmi les bases organiques, la méthylamine, la propylamine, la triméthylamine, la diéthylamine, la triéthylamine, la N,N-diméthyléthanolamine, le tris (hydroxyméthyl) amino méthane, l'éthanolamine, la pyridine, la picoline, la dicyclohexyl amine, la morpholine, la benzylamine, la procaïne, la lysine, l'arginine, l'histidine, la N-méthylglucamine.

Lorsque $R_1$ comporte une fonction salifiable par un acide et notamment une fonction amino, on obtient des sels d'addition avec les acides.

L'invention s'étend naturellement aux sels d'addition avec les acides des composés de formule (I) salifiables, comme, par exemple, les sels formés aveç les acides chlorhydrique, bromhydrique, nitrique, sulfurique, phosphorique, acétique, formique, propionique, benzoïque, maléïque, fumarique, succinique, tartrique, citrique, oxalique, glyoxylique, aspartique, alcane sulfoniques tels que les acides méthane ou éthane sulfoniques, arylsulfoniques, tels que les acides benzène ou paratoluène sulfoniques et arylcarboxyliques.

Parmi les produits de formule (I), on préfère les produits répondant à la formule (I') :

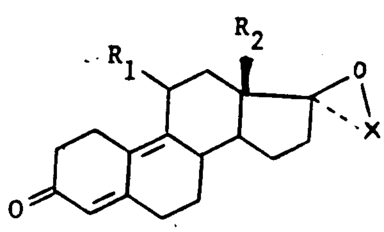

(I')

dans laquelle $R_1$ représente un radical aryle ou aralkyle carbocyclique ou hétérocyclique éventuellement substitué, $R_2$ en position béta, représente un radical hydrocarboné renfermant de 1 à 18 atomes de carbone, l'atome d'oxygène du spiro éther est en position 17béta, X représente un radical $-(CH_2)_n$ dans lequel n représente un entier égal à 2 ou 4 ou X représente un radical $-CH=CH-CH_2-CH_2-$ ainsi que leurs sels. Parmi les produits de formule (I) on préfère également les produits dans laquelle $R_1$ représente soit un radical aryle ou aralkyle portant une fonction amine :

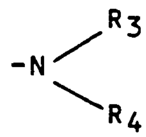

dans laquelle $R_3$ et $R_4$ représentent chacun un radical alkyle primaire, secondaire ou tertiaire renfermant de 1 à 8 atomes de carbone ou $R_3$ et $R_4$ forment avec l'atome d'azote auquel ils sont liés un hétérocycle comportant éventuellement un autre hétéroatome choisi dans le groupe formé par l'oxygène, l'azote, le soufre et le silicium, soit un radical aryle portant une fonction méthylthio ou éthylthio ainsi que leurs sels.

Parmi les derniers produits cités, on préfère particulièrement les produits de formule (I) dans laquelle $R_1$ est un radical phényle, le substituant porté par ce radical phényle est en position para, ainsi que leurs sels ainsi que les produits de formule (I) dans laquelle $R_1$ représente l'un des radicaux suivants :

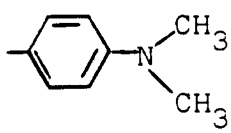 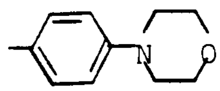 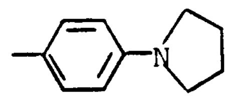 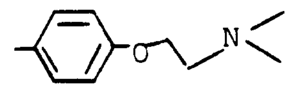

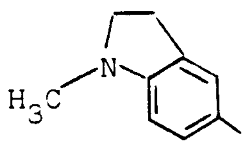 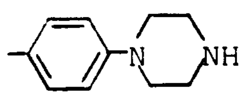 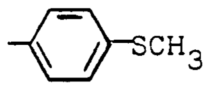 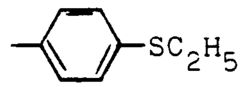

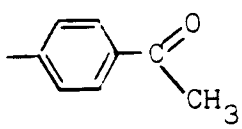 et 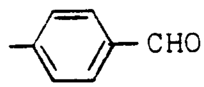

ainsi que leurs sels.

Parmi les valeurs de $R_1$ on préfère les radicaux :

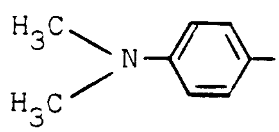 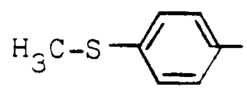

Les produits de formule (I) que l'on préfère sont particulièrement ceux décrits dans les exemples. Parmi ces produits décrits, on préfère :

- la (17R) 11béta-/4-(diméthylamino) phényl/ spiro (estra 4,9-dièn-17,2′-oxétan)-3-one.
- la (17R) 11béta-/4-(méthylthio)phényl/ spiro (estra 4,9-dièn-17,2′-oxétan)-3-one.

L'invention a également pour objet un procédé de préparation des produits de formule (I) telle que définie ci-dessus caractérisé en ce que :

a) pour préparer les produits de formule ($I_A$) :

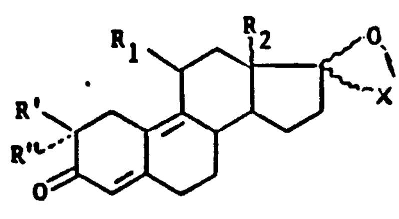

($I_A$)

dans laquelle $R_1$, X et $R_2$ conservant la même signification que ci-dessus R' et R" représentent chacun

un atome d'hydrogène ou un radical alkyle ou bien l'un représente un atome d'hydrogène et l'autre représente un radical alkyle, l'on soumet un produit de formule (II) :

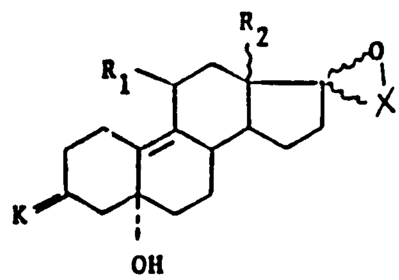

(II)

dans laquelle K représente un groupe cétonique bloqué à l'action d'un réactif de déshydratation également susceptible de libérer la fonction cétone pour obtenir les produits de formule ($I_A$) dans laquelle R' et R" représentent un atome d'hydrogène, et que, si désiré, l'on soumet à une oxydation les produits de formule ($I_A$) dans laquelle $R_1$ comporte un atome de soufre ou d'azote pour obtenir les produits dans lesquels $R_1$ comporte un atome de soufre oxydé en sulfoxyde ou en sulfone ou un atome d'azote oxydé en N-oxyde, et si désiré, soumet les produits de formule ($I_A$) à l'action d'une base forte puis d'un halogénure d'alkyle pour obtenir un produit de formule ($I_A$) dans laquelle R' et/ou R" représentent un radical alkyle ayant de 1 à 4 atomes de carbone ;

b) pour préparer les produits de formule ($I_B$) :

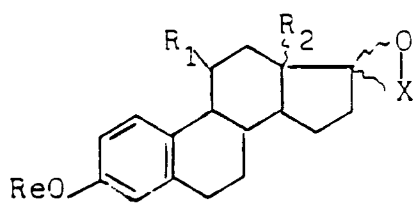

($I_B$)

dans laquelle $R_1$, $R_2$ et Re conservent la même signification que ci-dessus on soumet un produit de formule ($I'_A$) :

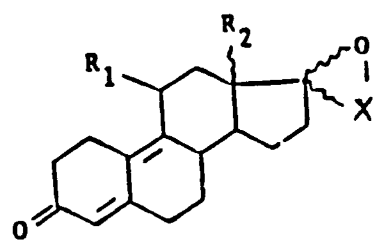

($I'_A$)

dans laquelle $R_1$ et $R_2$ ont la signification déjà indiquée, à l'action d'un agent d'aromatisation puis le cas échéant à l'action d'un agent de saponification et enfin si désiré soumet le produit de formule ($I_B$) dans laquelle Re est un atome d'hydrogène à un réactif d'alkylation ;

c) pour préparer les produits de formule ($I_C$) :

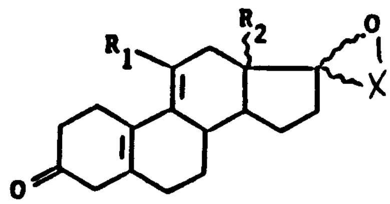

($I_C$)

dans laquelle $R_1$ et $R_2$ conservent la même signification que ci-dessus on soumet un produit de formule ($I'_A$) à l'action d'un agent d'acylation puis de saponification ;

d) pour préparer les produits de formule ($I_D$) :

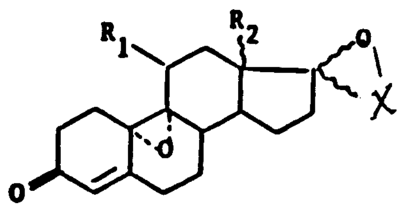

($I_D$)

dans laquelle $R_1$ et $R_2$ conservent la même signification que ci-dessus on soumet un produit de formule ($I'_A$) à l'action d'un agent d'époxydation ;

e) pour préparer les produits de formule ($I_E$) :

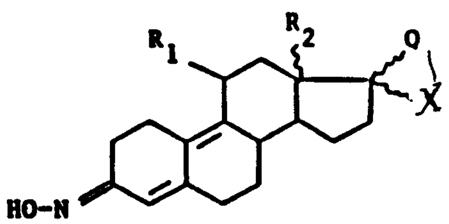

($I_E$)

dans laquelle $R_1$ et $R_2$ conservent la même signification que ci-dessus on fait agir l'hydroxylamine sur un produit de formule ($I'_A$).

f) l'on salifie, le cas échéant, les produits de formule $I_A$, $I_B$, $I_C$, $I_D$ et $I_E$ obtenus.

Dans un mode préférentiel d'exécution du procédé ci-dessus, la transformation des produits de formule II à l'aide d'un réactif de déshydratation également susceptible de libérer la fonction cétone, est effectuée de préférence à l'aide d'une résine sulfonique (forme acide) par exemple, une résine sulfonique du commerce à support de polystyrène ou à support de polymère styrène/divinyl/benzène. On peut cependant utiliser un acide minéral tel que l'acide chlorhydrique ou l'acide sulfurique dans un alcanol inférieur ou l'acide perchlorique dans l'acide acétique ou un acide sulfonique comme l'acide paratoluène sulfonique ou le sulfate acide de potassium dans le mélange eau/chlorure de méthylène.

Dans la formule II, K peut représenter un groupement cétal tel que diméthylcétal, éthylène dioxy, 2,2-diméthyl propan-1,3 dioxy, un thiocétal correspondant, un oxime ou un méthyloxime.

L'agent d'oxydation que l'on fait agir sur les produits de formule ($I_A$) ou l'agent d'époxydation que l'on fait agir pour obtenir les produits de formule ($I_D$) est de préférence un peracide tel que l'acide métachloroperbenzoïque, l'acide peracétique ou l'acide perphtalique. On peut également utiliser l'eau oxygénée seule ou en présence d'hexachloro ou d'hexafluoro acétone.

Bien entendu selon le nombre de fonctions pouvant faire l'objet d'une oxydation, on peut utiliser un ou plusieurs équivalents d'agent oxydant.

C'est ainsi, par exemple, que si l'on veut oxyder l'atome de soufre que comporte $R_1$, en sulfone, on doit bien entendu utiliser au moins deux équivalents d'agent d'oxydant.

- La base forte que l'on utilise sur les produits de formule ($I_A$) peut être un amidure de métal alcalin, tel que l'amidure de sodium ou de lithium éventuellement préparé in situ ;
- l'halogénure d'alkyle que l'on utilise est de préférence un iodure comme l'iodure de méthyle ;
- l'agent d'aromatisation utilisé pour préparer les produits de formule ($I_B$) est de préférence un halogénure d'acyle tel que le bromure d'acétyle ou un anhydride d'acide tel que l'anhydride acétique ou un mélange des deux ;
- l'acylation éventuelle des produits de formule ($I_B$) et l'acylation conduisant aux produits de formule ($I_C$) sont réalisées selon les méthodes usuelles ; on utilise de préférence un halogénure d'acyle ;
- l'alkylation éventuelle des produits de formule ($I_B$) est effectuée selon les méthodes usuelles. On utilise, par exemple, un halogénure d'alkyle ;
- l'agent de saponification que l'on utilise pour obtenir les produits de formule ($I_B$) ou ($I_C$) est de préférence une base alcaline comme la soude ou la potasse et la réaction est réalisée au sein d'un alcool inférieur tel que le méthanol ou l'éthanol ;
- l'oximation des produits de formule ($I'_A$) est effectuée en utilisant l'hydroxylamine sous forme de sel de préférence le chlorhydrate au sein d'un alcool à la température du reflux.

La salification est effectuée dans des conditions usuelles. On peut opérer, par exemple, en présence de soude éthanolique. On peut également utiliser un sel de sodium tel que le carbonate ou le carbonate acide de sodium ou de potassium.

De même, la salification par un acide est réalisée dans les conditions usuelles. On opère de préférence avec l'acide chlorhydrique, par exemple en solution éthérée.

L'invention a également pour objet un procédé de préparation des produits de formule générale (I) telle que définie ci-dessus dans laquelle X représente un groupement $-(CH_2)_4-$ ou $-CH=CH-CH_2-CH_2-$ caractérisé en ce que l'on soumet un produit de formule (III) :

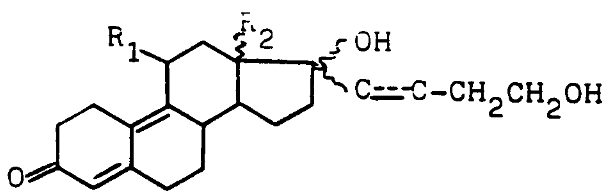

(III)

dans laquelle $R_1$ et $R_2$ conservent la même signification que ci-dessus et le trait pointillé indique la présence éventuelle d'une double liaison entre les carbones qui le portent, à l'action d'un réactif de cyclisation, pour obtenir un produit de formule ($I''_A$) :

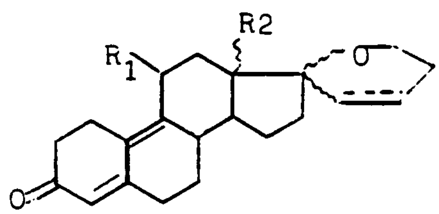

($I''_A$)

correspondant à un produit de formule ($I_A$) dans laquelle R' et R'' représentent chacun un atome d'hydrogène et X représente les groupements $-(CH_2)_4$ et $-CH=CH-CH_2-CH_2-$, produits de formule ($I''_A$) que l'on transforme en produits de formule ($I_A$) correspondants dans laquelle l'un des radicaux R' ou R'' ou les deux radicaux R' et R'' représentent un radical alkyle ayant de 1 à 4 atomes de carbone, et en produits de formules $I_B$, $I_C$, $I_D$ et $I_E$ selon le procédé décrit ci-dessus.

Dans un mode préférentiel d'exécution du procédé ci-dessus décrit, le réactif de cylisation que l'on fait agir de préférence sur les produits de formule (III) est le chlorure de tosyle en présence de pyridine ; on peut également utiliser le chlorure de méthylsulfonyle.

Les produits de formule (I) ainsi que leurs sels pharmaceutiquement acceptables sont des produits particulièrement intéressants du point de vue pharmacologique.

L'étude des produits sur les récepteurs hormonaux a permis de mettre en évidence des activités progestomimétiques ou antiprogestomimétiques, androgènes ou anti-androgènes.

Les produits de formule (I) possèdent en particulier une remarquable activité antiprogestomimétique. Les produits de formule (I) possèdent également une activité antiglucocorticoïde comme le montrent les résultats des test exposés ci-après.

Certains produits montrent cependant une activité anti progestomimétique supérieure à leur propriété antiglucocorticoïde.

Les produits de formule (I) ainsi que leurs sels pharmaceutiquement acceptables qui possèdent des propriétés antiprogestomimétiques peuvent être utilisés comme contraceptifs ; ils peuvent être utilisés contre les dérèglements hormonaux.

Certains produits de formule (I) ainsi que leurs sels pharmaceutiquement acceptables peuvent également présenter des propriétés progestomimétiques et peuvent ainsi être employées dans le traitement des aménorrhées, des dysménorrhées et des insuffisances lutéales.

Les produits de formule (I) ainsi que leurs sels pharmaceutiquement acceptables peuvent donc être utilisés comme médicaments pour lutter principalement contre les effets secondaires des glucocorticoïdes, ils permettent de lutter également contre les troubles dus à une hypersecrétion de glucocorticoïdes et notamment contre le vieillissement en général et plus particulièrement contre l'hypertension, l'athérosclérose, l'ostéoporose, le diabète, l'obésité ainsi que l'immunodépression et l'insomnie.

Les produits de formule (I) ainsi que leurs sels pharmaceutiquement acceptables qui présentent des propriétés antiandrogènes peuvent être utilisés dans le traitement des hypertrophies et du cancer de la prostate, de l'hyperandrogénie, de l'anémie, de l'hirsutisme et de l'acné.

Les produits de formule (I) ainsi que leurs sels pharmaceutiquement acceptables possèdent également des propriétés antiprolifératives qui les rendent utilisables dans le traitement des cancers hormono-dépendants notamment les carcinomes mammaires et leurs métastases. Ces propriétés les rendent également utilisables dans le traitement des tumeurs bénignes.

Certains des produits de formule (I) ainsi que leurs sels pharmaceutiquement acceptables possèdent des propriétés estrogènes et/ou anti-estrogènes. Les propriétés anti-estrogènes les rendent utilisables dans le traitement des cancers estrogèno-dépendants.

Les propriétés estrogènes que peuvent également présenter lesdits produits de formule (I) ainsi que leurs sels pharmaceutiquement acçeptables les rendent utilisables également dans le traitement des troubles liés à une hypofolliculinie, par exemple les aménorrhées, les dysménorrhées, les avortements répétés, les troubles prémenstruels ainsi que le traitement de la ménopause.

L'invention a donc pour objet à titre de médicament les produits de formule (I) pharmaceutiquement acceptables, c'est-à-dire non toxiques aux doses utilisées, ainsi que leurs sels pharmaceutiquement acceptables.

L'invention a particulièrement pour objet, à titre de médicaments, les produits de formule (I) préférés mentionnés plus haut et tout particulièrement les produits dont les noms suivent :

- la (17R) 11béta-/4-(diméthylamino) phényl/ spiro (estra 4,9-dièn-17,2′-oxétan)-3-one.
- la (17R) 11béta-/4-(méthylthio)phényl/ spiro (estra 4,9-dièn-17,2′-oxétan)-3-one.

La posologie utile varie en fonction de l'affection à traiter et de la voie d'administration ; elle peut varier par exemple de 10 mg à 1 g par jour chez l'adulte par voie orale.

Les nouveaux produits de formule (I) et leurs sels, tels que définis ci-dessus peuvent être employés pour préparer des compositions pharmaceutiques renfermant, à titre de principe actif, l'un au moins desdits produits.

Les produits de formule (I) et leurs sels sont utilisés par voie digestive, parentérale ou locale. Ils peuvent être prescrits ous forme de comprimés simples ou dragéifiés, de gélules, de granulés, de suppositoires et de préparations injectables, de pommades, de crèmes, de gels, lesquels sont préparés selon les méthodes usuelles.

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

L'invention a donc pour objet les compositions pharmaceutiques renfermant comme principe actif au moins un produit de formule (I) ou au moins un de leurs sels pharmaceutiquement acceptables.

L'invention a également pour objet à titre de produits industriels nouveaux et notamment à titre de produits industriels nouveaux nécessaires pour la préparation des produits de formule (I), les produits de formule II' :

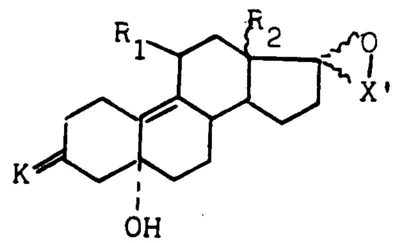

(II')

dans laquelle $R_1$, $R_2$ et K ont la signification indiquée ci-dessus et X' représente $-(CH_2)_{n1}-$ dans lequel $n_1$ = 2 ou 4 ou X' représente $-CH=CH-CH_2-CH_2-$.

Les produits de formule (II') peuvent être préparés par cyclisation des produits de formule (IV) correspondante :

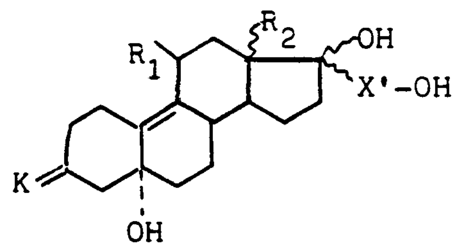

(IV)

lorsque X' représente $-(CH_2)_{n1}-$ dans lequel $n_1$ = 2 ou 4 ou lorsque X' représente $-CH=CH-CH_2-CH_2$.

Les produits de formule (IV) peuvent eux-mêmes être préparés à partir de produits de formule (V) :

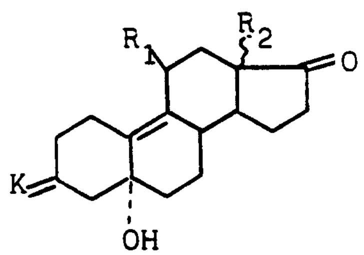

(V)

par action sur ces produits de dérivés de formules :
$H-C\equiv C-(CH_2)_2OH$ ou $CH_3CO_2Alk$ en présence d'une base forte, (Alk = alkyle $C_{1-4}$) suivie si nécessaire d'une séparation des mélanges d'isomères obtenus par les méthodes usuelles telles que la chromatographie.

Les produits obtenus dans lesquels le cycle D a la structure suivante :

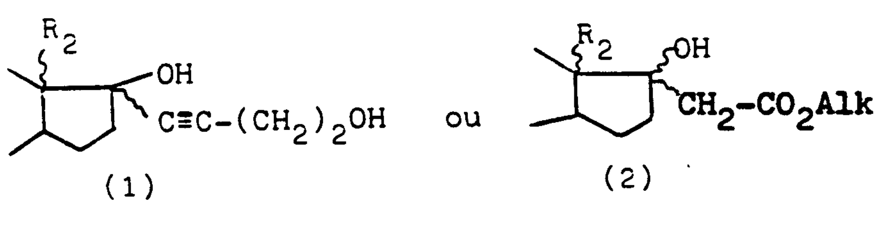

sont ensuite soumis à une hydrogénation partielle ou totale pour les premiers produits (1) pour obtenir les produits de formule (IV) dans laquelle X' représente respectivement un radical $-CH=CH-CH_2-CH_2-$ ou

$-(CH_2)_{n1}$ dans lequel $n_1$ représente le chiffre 4.

Les produits de formule (2) sont soumis à un réactif de réduction pour obtenir les produits de formule (IV) dans laquelle X′ représente $-(CH_2)_2-$.

Les produits de formule (II) dans laquelle X représente $-(CH_2)-$ peuvent être préparés par action sur les produits de formule (V) d'un halogénure de triméthyl sulfonium en présence d'une base forte.

On trouve des modes de préparation des produits de ce type dans le brevet européen EP 0.192.598.

Les produits de formule (II′) peuvent également être préparés de la facon suivante :

On fait agir sur un produit de formule (VI) :

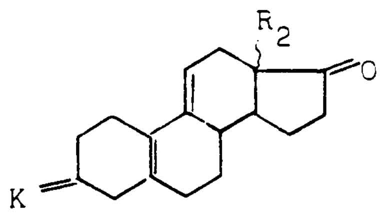

(VI)

soit un produit de formule : $H\text{-}C\equiv C\text{-}(CH_2)_2OH$ pour obtenir un produit ayant le même cycle D que le produit (1) ci-dessus et ensuite soumet le produit à une réaction d'hydrogénation partielle ou totale.

Soit un produit de formule : $CH_3CO_2Alk$ pour obtenir un produit ayant le même cycle D que le produit (2) ci-dessus et ensuite soumet ce produit à une réaction de réduction, pour obtenir un produit de formule (VII) :

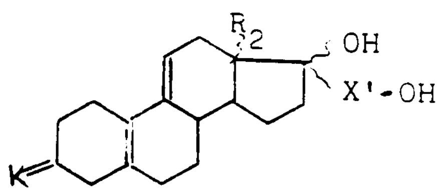

(VII)

dans laquelle X′ et $R_2$ ont la signification précédente et l'on soumet les produits de formule (VII) à un agent de cyclisation, pour obtenir un produit de formule (VIII) :

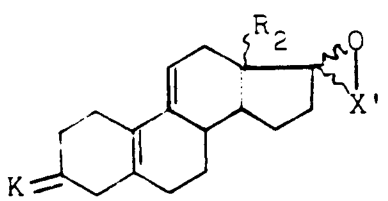

(VIII)

dans laquelle $R_2$ et X′ ont la signification précédente quel'on soumet à un réactif d'époxydation, pour obtenir un produit de formule (IX) :

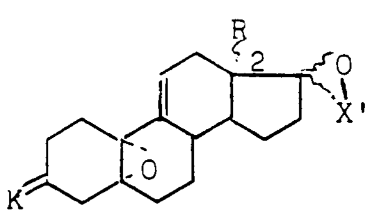

(IX)

produit sur lequel on fait agir un réactif choisi parmi les réactifs suivants : $(R_1)_2CuLi$, $R_1MgHal$ en présence de sels cuivreux ou cuivriques ou $(R_1)_2CuCNLi_2$, pour obtenir les produits de formule (II′) attendus.

Dans un mode préféré d'exécution du procédé ci-dessus, l'agent de cyclisation des produits de formule (IV) en produits de formule (II′) est le chlorure de toluène sulfonyle ou le chlorure de méthane sulfonyle dans la pyridine.

L'action du produit de formule : $H-C \equiv C-(CH_2)_2OH$ sur le produit de formule (V) est effectuée de préférence en présence de tert-butylate de potassium ou de butyl ou méthyl lithium dans un solvant tel que le tétrahydrofuranne.

L'action du produit de formule : $CH_3CO_2Alk$ dans laquelle Alk est de préférence le tert-butyle est effectuée de préférence en présence de diisopropylamidure de lithium dans un solvant tel que le tétrahydrofuranne.

L'hydrogénation totale du produit (1) est effectuée par exemple par le mélange méthanol palladium sur charbon ou par le chlorotristriphényl phosphino rhodium dans le benzène ou le mélange benzène/éthanol et passage d'hydrogène. L'hydrogénation partielle peut être effectuée par l'hydrogène en présence de palladium sur sulfate de baryum empoisonné par une amine telle que la quinoléine ou la triéthylamine, dans un solvant tel que l'acétate d'éthyle.

La réduction des produits (2) peut être effectuée de préférence à l'aide d'hydrure de lithium aluminium par exemple dans le tétrahydrofuranne.

La formation de l'époxyde en position 17,20 est effectuée de préférence par action de l'iodure de triméthylsulfonium en présence de tert butylate de potassium dans un solvant ou un mélange de solvants tels que tétrahydrofuranne et diméthyl sulfoxyde.

Les produits de formule (III) peuvent être préparés par action sur les produits de formule (IV) précités d'un réactif de déshydratation également susceptible de libérer la fonction cétone choisis parmi les réactifs cités ci-dessus et notamment l'acide acétique dilué ou l'acide chlorhydrique dans le méthanol.

Cette réaction peut d'ailleurs être effectuée à partir des produits (I) ci-dessus sans isolement du produit (IV).

La réaction d'hydrogénation partielle ou totale des produits (I) étant suivie de la réaction de déprotection de la fonction cétone.

Le réactif d'époxydation utilisé pour transformer les produits de formule (VIII) en produits de formule (IX) est de peroxyde d'hydrogène dans l'hexafluoroacétone. On sépare l'isomère béta non recherché par chromatographie.

Le réactif que l'on fait agir de préférence sur le produit de formule (IX) est le produit $R_1MgBr$ en présence de chlorure cuivreux.

On trouvera ci-après dans la partie expérimentale des exemples de réalisation de telles préparations.

En plus des exemples suivants qui illustrent l'invention sans toutefois la limiter, les produits suivants constituent des produits pouvant être obtenus dans le cadre de la présente invention.

Les produits de formule ($I'_A$) :

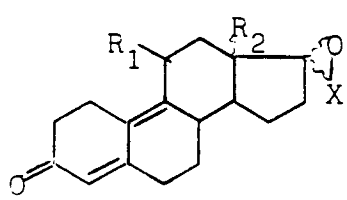

($I'_A$)

dans laquelle $R_1$, $R_2$ et X ont la valeur suivante et l'atome d'oxygène du piro éther est en position 17béta.

| $R_1$ | $R_2$ | X |
|---|---|---|
| 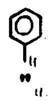 | β $CH_3$ | $-CH_2$ |
| " | " | $(CH_2)_2$ |
| " | " | $(CH_2)_4$ |
| " | " | $CH=CH-CH_2-CH_2$ |
| 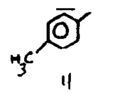 | " | $-CH_2$ |
| " | " | $(CH_2)_2$ |
| " | " | $(CH_2)_4$ |
| " | " | $CH=CH-CH_2-CH_2$ |
| 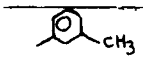 | " | $-CH_2$ |
| " | " | $(CH_2)_2$ |
| " | " | $(CH_2)_4$ |
| " | " | $CH=CH-CH_2-CH_2$ |
| 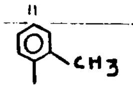 | " | $-CH_2$ |
| " | " | $(CH_2)_2$ |
| " | " | $(CH_2)_4$ |
| " | " | $CH=CH-CH_2-CH_2$ |
| 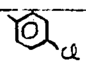 | " | $-CH_2$ |
| " | " | $(CH_2)_2$ |
| " | " | $(CH_2)_4$ |
| " | " | $CH=CH-CH_2-CH_2$ |
| 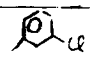 | " | $-CH_2$ |
| " | " | $(CH_2)_2$ |
| " | " | $(CH_2)_4$ |
| " | " | $CH=CH-CH_2-CH_2$ |
| 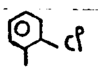 | " | $-CH_2$ |
| " | " | $(CH_2)_2$ |
| " | " | $(CH_2)_4$ |
| " | " | $CH=CH-CH_2-CH_2$ |
| 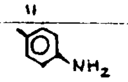 | " | $-CH_2$ |
| " | " | $(CH_2)_2$ |
| " | " | $(CH_2)_4$ |
| " | " | $CH=CH-CH_2-CH_2$ |
| 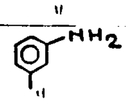 | " | $-CH_2$ |
| " | " | $(CH_2)_2$ |
| " | " | $(CH_2)_4$ |
| " | " | $CH=CH-CH_2-CH_2$ |

| $R_1$ | $R_2$ | X |
|---|---|---|
| 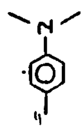 | β $C_2H_5$ | $-CH_2$ |
| " | " | $(CH_2)_2$ |
| " | " | $(CH_2)_4$ |
| " | " | $CH=CH-CH_2-CH_2$ |
| " | α $CH_3$ | $-CH_2$ |
| " | " | $(CH_2)_2$ |
| " | " | $(CH_2)_4$ |
| " | " | $CH=CH-CH_2-CH_2$ |
| 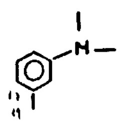 | β $CH_3$ | $-CH_2$ |
| " | " | $(CH_2)_2$ |
| " | " | $(CH_2)_4$ |
| " | " | $CH=CH-CH_2-CH_2$ |
| 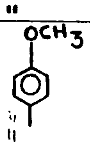 | " | $-CH_2$ |
| " | " | $(CH_2)_2$ |
| " | " | $(CH_2)_4$ |
| " | " | $CH=CH-CH_2-CH_2$ |
| 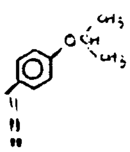 | " | $-CH_2$ |
| " | " | $(CH_2)_2$ |
| " | " | $(CH_2)_4$ |
| " | " | $CH=CH-CH_2-CH_2$ |
| 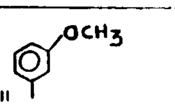 | " | $-CH_2$ |
| " | " | $(CH_2)_2$ |
| " | " | $(CH_2)_4$ |
| " | " | $CH=CH-CH_2-CH_2$ |
| 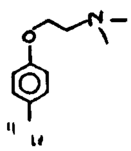 | " | $-CH_2$ |
| " | " | $(CH_2)_2$ |
| " | " | $(CH_2)_4$ |
| " | " | $CH=CH-CH_2-CH_2$ |
| 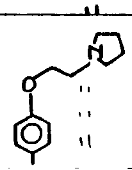 | " | $-CH_2$ |
| " | " | $(CH_2)_2$ |
| " | " | $(CH_2)_4$ |
| | " | $CH=CH-CH_2-CH2$ |
| 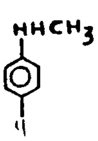 | " | $-CH_2$ |
| " | " | $(CH_2)_2$ |
| " | " | $(CH_2)_4$ |
| " | " | $CH=CH-CH_2-CH_2$ |

| $R_1$ | $R_2$ | X |
|---|---|---|
| 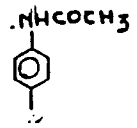 | β-$CH_3$ | $-CH_2$ |
| | " | $(CH_2)_2$ |
| " | " | $(CH_2)_4$ |
| " | " | $CH=CH-CH_2-CH_2$ |
| 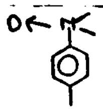 | " | $-CH_2$ |
| | " | $(CH_2)_2$ |
| " | " | $(CH_2)_4$ |
| 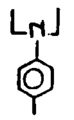 | " | $-CH_2$ |
| | " | $(CH_2)_2$ |
| " | " | $(CH_2)_4$ |
| " | " | $CH=CH-CH_2-CH_2$ |
| 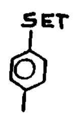 | " | $-CH_2$ |
| | " | $(CH_2)_2$ |
| " | " | $(CH_2)_4$ |
| " | " | $CH=CH-CH_2-CH_2$ |
| 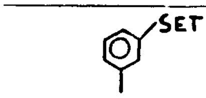 | " | $-CH_2$ |
| | " | $(CH_2)_2$ |
| " | " | $(CH_2)_4$ |
| " | " | $CH=CH-CH_2-CH_2$ |
| 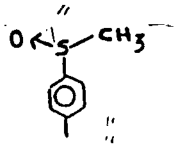 | " | $-CH_2$ |
| | " | $(CH_2)_2$ |
| " | " | $(CH_2)_4$ |
| 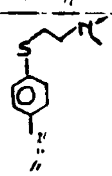 | " | $-CH_2$ |
| | " | $(CH_2)_2$ |
| " | " | $(CH_2)_4$ |
| " | " | $CH=CH-CH_2-CH_2$ |
| 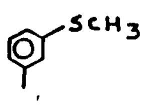 | | $-CH_2$ |
| | " | $(CH_2)_2$ |
| | " | $(CH_2)_4$ |
| " | " | $CH=CH-CH_2-CH_2$ |
| 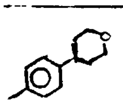 | " | $-CH_2$ |
| | " | $(CH_2)_2$ |
| | " | $(CH_2)_4$ |
| " | " | $CH=CH-CH_2-CH_2$ |

| $R_1$ | $R_2$ | X |
|---|---|---|
| 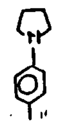 | β-$CH_3$ | $-CH_2$ |
| | " | $(CH_2)_2$ |
| | " | $(CH_2)_4$ |
| | " | $CH=CH-CH_2-CH_2$ |
| 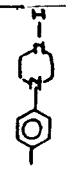 | " | $-CH_2$ |
| | " | $(CH_2)_2$ |
| | " | $(CH_2)_4$ |
| | " | $CH=CH-CH_2-CH_2$ |
| 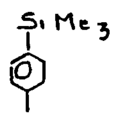 | " | $-CH_2$ |
| | " | $(CH_2)_2$ |
| | " | $(CH_2)_4$ |
| | " | $CH=CH-CH_2-CH_2$ |
| 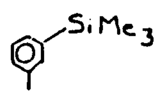 | " | $-CH_2$ |
| | " | $(CH_2)_2$ |
| | " | $(CH_2)_4$ |
| | " | $CH=CH-CH_2-CH_2$ |
| 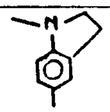 | " | $-CH_2$ |
| | " | $(CH_2)_2$ |
| | " | $(CH_2)_4$ |
| | " | $CH=CH-CH_2-CH_2$ |
| 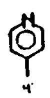 | " | $-CH_2$ |
| | " | $(CH_2)_2$ |
| | " | $(CH_2)_4$ |
| | " | $CH=CH-CH_2-CH_2$ |
| 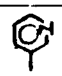 | " | $-CH_2$ |
| | " | $(CH_2)_2$ |
| | " | $(CH_2)_4$ |
| | " | $CH=CH-CH_2-CH_2$ |
| 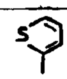 | " | $-CH_2$ |
| | " | $(CH_2)_2$ |
| | " | $(CH_2)_4$ |
| | " | $CH=CH-CH_2-CH_2$ |
| 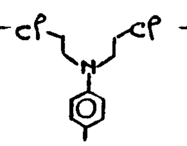 | " | $-CH_2$ |
| | " | $(CH_2)_2$ |
| | " | $(CH_2)_4$ |
| | " | $CH=CH-CH_2-CH_2$ |

| $R_1$ | $R_2$ | X |
|---|---|---|
| 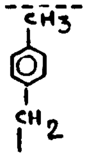 | " | $-CH_2$ |
| | " | $(CH_2)_2$ |
| | " | $(CH_2)_4$ |
| | | $CH=CH-CH_2-CH_2$ |
| 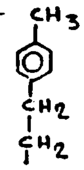 | " | $-CH_2$ |
| | " | $(CH_2)_2$ |
| | | $(CH_2)_4$ |
| | " | $CH=CH-CH_2-CH_2$ |
| 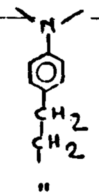 | " | $-CH_2$ |
| | | $(CH_2)_2$ |
| | " | $(CH_2)_4$ |
| | | $CH=CH-CH_2-CH_2$ |
| 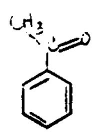 | " | $-CH_2$ |
| | | $(CH_2)_2$ |
| | " | $(CH_2)_4$ |
| | | $CH=CH-CH_2-CH_2$ |
| 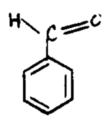 | " | $-CH_2$ |
| | | $(CH_2)_2$ |
| | | $(CH_2)_4$ |
| | " | $CH=CH-CH_2-CH_2$ |
| 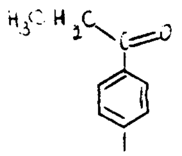 | " | $-CH_2$ |
| | " | $(CH_2)_2$ |
| | " | $(CH_2)_4$ |
| | " | $CH=CH-CH_2-CH_2$ |

**Exemple 1 : (17R) 11béta-/4-(diméthylamino) phényl/ spiro (estra-4,9-dien-17,2'-oxetan)-3-one.**

Stade A : 3,3-[(1,2-éthanediyl) bis oxy]17béta-hydroxy 19-nor 17alpha-pregna-5(10), 9(11)-dièn-21-oate de (1,1-diméthyl éthyle).

On refroidit à -73 °C une solution de 568 cm3 de diisopropylamidure de lithium titrant 0,51M dans 1,4 litres de tétrahydrofuranne, introduit en 20 minutes à -73 °C/-72 °C, 37,3 cm3 d'acétate de terbutyle et agite une heure à -73 °C. On laisse remonter la température à -60 °C, introduit en trente minutes 17,38 g de 3,3-(1,2-éthanediyl) acétal cyclique d'estra-5(10),9(11)-dièn-3,17-dione dans 260 cm3 de tétrahydrofuranne et agite ensuite une heure 20 minutes à -60 °C.

On amène la température du milieu à 0 °C et ajoute en 15 minutes 520 cm3 de chlorure d'ammonium en solution aqueuse saturée, agite une heure à température ambiante, décante et extrait la phase aqueuse au chlorure de méthylène. On lave les phases organiques au chlorure de sodium en solution aqueuse saturée, sèche puis amène à sec. On chromatographie le résidu sur silice, élue par un mélange cyclohexane-acétate d'éthyletriéthylamine 95-5-1‰. On isole 20,3 g de produit attendu.

Spectre RMN ($CDCl_3$ + 1 goutte $C_5D_5N$)

3,97 ppm : H éthylène dioxy en 3
5,6 ppm : $H_{11}$
0,9 ppm : $H_{18}$
4,55 ppm : OH
1,47 ppm : H du tBu
Préparation du diisopropylamidure de lithium selon House et coll. J. Org. Chem. 43, 704 (1978).
A partir de 68 cm3 de diisopropylamine, on a obtenu le produit attendu en solution : titre 0,51M.

Stade B : (1,2-éthanediyl) acétal cyclique de 17,21-dihydroxy 19-nor 17alpha-pregna-5(10),9(11)-dièn-3-one.

On dissout sous atmosphère inerte, 20,3 g de produit obtenu ci-dessus dans 400 cm3 de tétrahydrofuranne et ajoute en 5 minutes à +18°C/+22°C 14,38 g d'hydrure de lithium-aluminium. On chauffe à 40°C pendant une heure. Après refroidissement à +15°C/+20°C on ajoute 600 cm3 de chlorure de méthyléne, amène à 0°C/+5°C et ajoute lentement 130 cm3 de solution aqueuse saturée en bicarbonate de soude. On filtre leprécipité obtenu, le lave par empâtage au chlorure de méthylène, décante le filtrat et extrait la phase aqueuse au chlorure de méthylène. On lave les phases organiques au chlorure de sodium en solution aqueuse saturée, sèche et amène à sec. On empâte à l'éther le résidu, essore, sèche sous pression réduite et obtient 12,47 g de produit attendu.

Spectre RMN ($CDCl_3$ + 1 goutte $C_5D_5N$)

3,96 ppm : H de l'éthylène dioxy
5,59 ppm : $H_{11}$
0,88 ppm : $H_{18}$
3,97 ppm : $H_{21}$
3,33 ppm : H des OH

Stade C : (17R) (1,2-éthanediyl) acétal cyclique de spiro(estra-5(10),9(11)-dièn-17,2'-oxétan)-3-one.

On dissout 12,47 g du produit obtenu ci-dessus dans 250 cm3 de pyridine. On ajoute peu à peu 24,94 g de chlorure de tosyle et agite deux heures à température ambiante. On ajoute de la glace, de l'eau, extrait à l'acétate d'éthyle, lave les phases organiques avec une solution aqueuse saturée en chlorure de sodium, sèche et amène à sec par entraînements au toluène. On met en suspension les 19,80 g de tosylate obtenu dans 890 cm3 de potasse éthanolique à 5% et agite au reflux pendant 40 minutes. On distille l'éthanol sous pression réduite, ajoute de l'eau et de la glace, agite 10 minutes, essore le précipité, le lave à l'eau jusqu'à neutralité, le dissout dans le chlorure de méthylène, décante l'eau, sèche et amène à sec. On chromatographie sur silice le résidu (éluant cyclohexanne-acétate d'éthyle-triéthylamine 90-10-1‰ et isole 8,97 g de produit attendu. F = 136°C.

Spectre RMN ($CDCl_3$) ppm

3,99 : H de l'éthylène dioxy
5,64 : $H_{11}$
0,76 : $H_{18}$
4,30 à 4,50 : $H_{21}$

Stade D : (17R) (1,2-éthanediyl) acétal cyclique de 5alpha,10alpha-époxy spiro(estra-5(10),9(11-dièn-17,2'-oxétan)-3-one et son isomère 5béta,10béta-époxy.

On dissout les 8,97 g de produit obtenu précédemment dans 180 cm3 de chlorure de méthylène, ajoute 9 cm3 d'hexachloroacétone, refroidit à -30°C et introduit en 15 minutes à -33°C/-31°C 27 cm3 d'eau oxygénée à 50% puis on agite 3 heures à 0°/+2°C. On verse le mélange réactionnel sur 300 cm3 d'une solution saturée en bicarbonate de soude, ajoute à +15°C/+20°C du thiosulfate de sodium jusqu'à absence de peroxyde et décante. On extrait la phase aqueuse au chlorure de méthylène, lave les phases organiques à l'eau et avec une solution aqueuse saturée en chlorure de sodium, sèche et amène à sec. On chromatographie sur silice le résidu (éluant : cyclohexanne - acétate d'éthyle - triéthylamine 80-20-1‰ et

isole 1,82 g de produit 5béta,10béta-époxy et 5,36 g de produit 5alpha,10alpha-époxy.

Spectre IR ($CHCl_3$)

| | |
|---|---|
| époxy alpha | : 973 $cm_{-1}$ |
| | : 823 $cm_{-1}$ |
| époxy béta | : 981 $cm_{-1}$ |
| | : 830 $cm_{-1}$ |

Spectre RMN ($CDCl_3$)

| | |
|---|---|
| époxy alpha : | 0,76 $H_{18}$ |
| | 3,92 ppm cétal |
| | 6,09 ppm $H_{11}$ |
| | : 4,30 à 4,46 ppm $H_{21}$ |
| époxy béta | : 0,75 ppm $H_{18}$ |
| | : 3,91 ppm cétal |
| | : 5,92 ppm $H_{11}$ |
| | : 4,30 à 4,46 ppm $H_{21}$ |

Stade E : (17R) (1,2-éthanediyl) acétal cyclique de 5alpha-hydroxy 11béta-/4-(diméthylamino)phényl/spiro-(estr-9-èn-17,-2'-oxétan)-3-one.

On dissout sous atmosphère inerte, 0,861 g d'époxyde 5alpha-10alpha obtenu ci-dessus dans 18 cm3 de tétrahydrofuranne, ajoute 24 mg de chlorure cuivreux, refroidit à 0 ° C/ + 5 ° C, introduit en 30 minutes, 8,3 cm3 de bromure de 4-diméthylaminophényl magnésium en solution (0,87M) dans du tétrahydrofuranne et agite une heure à cette température. On verse le mélange réactionnel à + 15 ° C/ + 20 ° C sur 40 cm3 de chlorure d'ammonium en solution aqueuse saturée, agite 10 minutes, décante, extrait la phase aqueuse à l'acétate d'éthyle. On lave les phases organiques avec une solution saturée en chlorure de sodium, sèche et amène à sec. On chromatographie le résidu sur silice (éluant : cyclohexanne - acétate d'éthyletriéthylamine 70-30-1‰. On isole 1 g de produit attendu. F = 178 ° C.

Spectre RMN ($CDCl_3$)

4,00 ppm : H éthylène dioxy
2,91 ppm : H diméthylamino
4,28 à 4,45 ppm : $H_{11}$ et $H_{21}$
0,40 ppm : $H_{18}$
4,31 ppm : OH

Stade F : (17R) 11béta-/4-(diméthylamino)phényl/ spiro(estra-4,9-dièn-17,2'-oxétan)-3-one.

On dissout 0,944 g de produit préparé ci-dessus dans 6,6 cm3 d'acide acétique à 70%, chauffe à 41 ° C/43 ° C et agite deux heures 15 minutes. On refroidit à 0 ° C/ + 5 ° C, ajoute de l'ammoniaque jusqu'à pH supérieur à 8, extrait au chlorure de méthylène, lave avec une solution saturée en chlorure de sodium et amène à sec. On chromatographie le résidu sur silice (éluant cyclohexanne - acétate d'éthyle 80-20) et isole 0,468 g de produit attendu.
Après recristallisation dans l'isopropanol le produit fond à 108 ° C.

Spectre IR ($CHCl_3$)

| | |
|---|---|
| cétone conjuguée : | 1654 $cm^{-1}$ |
| C = C | 1612 $cm^{-1}$ |
| aromatique : | 1561 $cm^{-1}$ |

Spectre U.V. (EtOH)

Max. 260 nm ε = 17.400
Max. 302 nm ε = 20.500

EtOH HCl 0,1N

Max. 300 nm ε = 18.900
Infl. 218 - 240 nm.

Spectre RMN ($CDCl_3$) ppm

2,92 : H diméthylamino
5,76 : $H_4$
0,48 : $H_{18}$
4,40 : $H_{11}$ et $H_{21}$

~ 6,67 / 7,07 } aromatiques.

**Exemple 2 : (17R) 11béta-/4-(méthylthio)phényl/ spiro-(estra-4,9-dièn-17,2′-oxétan)-3-one.**

Stade A : (17R) (1,2-éthanediyl) acétal cyclique de 5alpha-hydroxy 11béta-/4-(méthylthio)phényl/ spiro(estr-9-èn-17,2′-oxétan)-3-one.

On refroidit à 0°C/+5°C pendant 15 minutes sous atmosphère inerte 55,3 cm3 de 4-(méthylthio) phényl magnésium dans le tétrahydrofuranne (titre 0,81M) et 0,148 g de chlorure cuivreux. On introduit en 25 minutes à +3,5°C/+5°C, 5,36 g de (17R) (1,2-éthane diyl) acétal cyclique de 5alpha,10alpha-époxy spiro(estra-5(10),9(11)-dièn-17,2′-oxétan)-3-one dans 53 cm3 de tétahydrofuranne et agite à 0°C/+5°C pendant une heure et demie puis on opère comme au stade E de l'exemple 1 pour obtenir 0,24 g de produit attendu.

Spectre RMN ($CDCl_3$)

2,46 ppm :$H_3C$-S
3,9 à 4,1 ppm :H éthylène dioxy
0,38 ppm :$H_{18}$
4,30 à 4,45 ppm :$H_{11}$, $H_{21}$ et OH
7,16 ppm:aromatiques.

Stade B : (17R) 11béta-/4-(méthylthio)phényl/spiro-(estra-4,9-dièn-17,2′-oxétan)-3-one.

On dissout 2 g du produit obtenu comme ci-dessus dans 40 cm3 d'éthanol, ajoute 2 g de résine Amberlite IRC 84 (Rohm Haas) et chauffe à reflux 7 heures sous atmosphère inerte. A température ambiante, on essore le mélange résine plus produits, rince à l'éthanol, au chlorure de méthylène et amène à sec le filtrat pour obtenir 1,194 g de produit attendu. F = 154°C.
Après 2 recristallisations dans l'éthanol 99,8%, on obtient un produit fondant à 160°C.

Spectre IR ($CHCl_3$)

C=O : 1653 $cm^{-1}$
C=C : 1603 $cm^{-1}$

aromatiques { 1556 $cm^{-1}$
1493 $cm^{-1}$ }

Spectre U.V. (EtOH)

Max. 260 nm , $\epsilon$ = 16.500
Max. 300 nm , $\epsilon$ = 20.100

Spectre RMN ($CDCl_3$)

5,77 ppm : $H_4$
2,46 ppm : S-$CH_3$
0,44 ppm : $H_{18}$
4,3 à 4,5 ppm : $H_{21}$

7,12 ppm
7,18 ppm } : aromatiques

**Exemple 3 : (17S) 11béta-/4-(diméthylamino)phényl/3',4',5',6'-tétrahydro spiro(estra-4,9-dièn-l7,2'-(2H) pyran)-3-one.**

Stade A : (1,2-éthane diyl) acétal cyclique de 5alpha,17béta-dihydroxy 11béta-/4-(diméthylamino)-phényl/17alpha-(4-hydroxy 1-butynyl) estr-9-èn-3-one.

On dissout 5 g de terbutylate de potassium dans 20 cm3 de tétrahydrofuranne anhydre, refroidit à -5°C et introduit en 5 minutes 1,9 g de (1,2-éthane diyl) acétal cyclique de 5alpha-hydroxy 11béta-/4-(diméthylamino)phényl/ estr-9-èn-3-one. On agite 10 minutes puis introduit en 15 minutes une solution de 2,4 cm3 de 3-butynol dans 20 cm3 de tétrahydrofuranne, agite pendant une heure et verse dans 200 cm3 d'une solution saturée en phosphate monosodique puis extrait la phase aqueuse au chlorure de méthylène, sèche et amène à sec. On purifie le résidu sur silice en éluant par un mélange cyclohexanne - acétate d'éthyle (1-1) puis par l'acétate d'éthyle à 1% d'ammoniaque. On récupère 1,775 g de produit attendu.

Spectre RMN ($CDCl_3$ + 1 goutte $C_5D_5N$)

2,90 ppm : H du diméthylamino
3,97 ppm : H éthylène dioxy
0,5 ppm : $H_{18}$
3,73 ppm : $CH_2$-OH
6,62 et 7,04 ppm : aromatiques.

Stade B : 11béta-/4-(diméthylamino)phényl/17béta-hydroxy 17alpha-(4-hydroxy butyl) estra-4,9-dièn-3-one.

On hydrogène 871 mg du produit obtenu ci-dessus dans 40 cm3 de méthanol en présence de 0,4 g de palladium/charbon. Après filtration, élimination des solvants, on reprend le résidu dans 10 cm3 d'acide acétique à 10% et agite pendant une heure à 45°C. On neutralise par une solution aqueuse saturée au bicarbonate de sodium puis extrait au chlorure de méthylène. On sèche la phase organique, amène à sec sous pression réduite. On chromatographie le résidu sur silice, élue à l'acétate d'éthyle et obtient 640 mg de produit attendu.

Spectre IR ($CHCl_3$)

C = O 1654 $cm^{-1}$

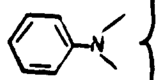

1612 $cm^{-1}$
1561 $cm^{-1}$
1518 $cm^{-1}$

OH : 3615 $cm^{-1}$

Spectre RMN ($CDCl_3$)

2,9 ppm :

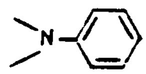

4,33 ppm $H_{11}$
0,57 ppm $H_{18}$
5,73 $H_4$
3,67 $CH_2OH$
6,65 et 7,02 ppm aromatiques

Stade C : (17S) 11béta-/4-(diméthylamino)phényl/ 3′,4′,5′6′-tétrahydro spiro(estra-4,9-dièn-17,2′(2H) pyran)-3-one.

On refroidit à 0 ° C une solution renfermant 788 mg de produit obtenu au stade précédent et 15 cm3 de pyridine anhydre, ajoute 1,5 g de chlorure de tosyle et agite pendant une heure à température ambiante. On agite 30 cm3 de glace puis neutralise par 14,5 cm3 d'acide chlorhydrique concentré. On extrait la phase aqueuse au chlorure de méthylène, sèche et amène à sec. On purifie le résidu par passage sur silice, éluant chlorure de méthylène - acétone 95-5 et récupère 0,2 g de produit attendu.

Spectre RMN ($CDCl_3$)

2,90 ppm : H du diméthylamino
4,33 ppm : $H_{11}$
0,53 ppm : $H_{18}$
3,46 et 3,74 ppm : $-CH_2O$
5,74 ppm : $H_4$
7,02 et 6,64 ppm : aromatiques

**Exemple 4 : (17S) 11béta-/4-(diméthylamino)phényl/spiro (estra-4,9-dièn-17,2′-oxiran)-3-one.**

On opère comme au stade B de l'exemple 2 à partir de 2,42 g de (1,2-éthane diyl) acétal cyclique de 11béta-/4-(diméthyl amino)phényl/ 5alpha-hydroxy spiro(estr-9-èn-17,2′-oxiran)-3-one avec la résine Amberlite IRC 84.

Après refroidissement on filtre la résine, la rince avec 60 cm3 d'éthanol et amène à sec. On empâte le résidu dans 30 cm3 d'éther isopropylique en agitant à 40 ° C. On glace, essore, rince à l'éther isopropylique, sèche sous pression réduite et obtient 0,844 g de produit attendu. Des liqueurs-mères on récupère encore 0,273 g de produit. F = 228 ° C

Après purification par chromatographie sur silice éluant cyclohexanne - acétate d'éthyle 70-30, on isole 0,750 g de produit du 1er jet. F = 234 ° C.
$/alpha/_D$ = + 270 ° ± 3,5 ° (c = 0,5% $CHCl_3$)

Spectre RMN ($CDCl_3$)

2,92 ppm : H du diméthylamino
5,8 ppm : $H_4$
4,33 ppm : $H_{11}$
0,6 ppm : $H_{18}$
2,63 et 2,98 ppm : H de l'oxirane
6,67 et 7,01 ppm : aromatiques

**Exemple 5 : (17S) 11béta-/4-(méthylthio)phényl/ spiro-(estra-4,9-dièn-17,2′-oxiran)-3-one.**

Stade A : 3,3-(1,2-éthanediyl) acétal cyclique de 5alpha-hydroxy 11béta-/4-(méthylthio)phényl/ estr-9-èn-3,17-diène.

On refroidit à -6°C, 0,672 g de chlorure cuivrique 0,212 g de chlorure de lithium, 165 cm3 de tétrahydrofuranne anhydre et 16,5 g de 3,3-(1,2-éthanediyl) acétal cyclique de 5alpha, 10alpha-époxy estr-9-(11)èn-3,17-dione et introduit goutte à goutte en une heure 15 minutes, 100 cm3 d'une solution 0,75M de bromure de 4-(méthylthio) phényl magnésium. On agite sous atmosphère inerte à -10°C pendant une heure, ajoute 100 cm3 d'une solution saturée en chlorure d'ammonium et agite 10 minutes.
On extrait à l'acétate d'éthyle, lave à l'eau, sèche et concentre à sec sous pression réduite. On reprend le résidu par un peu d'hexane, essore, lave l'hexane et obtient après séchage sous pression réduite à 60°C pendant une heure 22,2 g de produit brut. F = 202°C.
Après chromatographie sur silice de celui-ci, éluant cyclohexanne - acétate d'éthyle 1/1 + 1‰ triéthylamine, recristallisation dans l'acétate d'éthyle, on obtient le produit fondant à 209°C.

Stade B : (17S) (1,2-éthanediyl) acétal cyclique de 11béta-/4-(méthylthio) phényl/ spiro(estr-9-èn-17,2′-oxiran)-3-one.

On refroidit à 5°C, 12,7 cm3 d'une solution (0,9M) de terbutylate de potassium, 22 cm3 de tétrahydrofuranne, 27 cm3 de diméthylsulfoxyde anhydre, ajoute en quelques minutes 2,20 g d'iodure de triméthyl sulfonium et agite 30 minutes à 5°C. On verse ensuite à 5° C ± 1° en 7 minutes 2,45 g du produit obtenu ci-dessus dans 30 cm3 de tétrahydrofuranne anhydre et agite une heure à 0°C/+5° C. On verse le mélange réactionnel sur 300 cm3 d'eau, extrait à l'acétate d'éthyle, lave avec une solution aqueuse saturée en chlorure de sodium, sèche et amène à sec. On reprend le résidu par 15 cm3 d'éther éthylique, glace, essore, empâte avec un minimum d'éther éthylique froid, sèche sous pression réduite et obtient 2,1 g de produit attendu. F = 130°C (inst.)

Spectre IR ($CHCl_3$)

| | |
|---|---|
| OH : | 3508 $cm^{-1}$ |
| Aromatiques : | 1592 $cm^{-1}$ |
| | 1555 $cm^{-1}$ |
| | 1493 $cm^{-1}$ |

Stade C :(17S) 11béta-/4-(méthylthio)phényl/ spiro-(estra-4,9-dièn-17,2′-oxiran)-3-one.

On dissout sous atmosphère inerte 2,1 g du produit préparé ci-dessus dans 100 cm3 d'éthanol. On ajoute 4,2 g de résine Amberlite IRC 84 (RHOM-HAAS) et porte au reflux pendant 15 heures 30 minutes. Après refroidissement, on filtre, lave à l'éthanol, évapore le filtrat, maintient une heure au froid, essore et empâte le produit avec un minimum d'éthanol froid, sèche sous pression réduite et isole 0,49 g de produit attendu. F = 121-122°C.
Après recristallisation dans un mélange éthanol - chlorure de méthylène 4-5, on obtient un produit fondant à 122°C puis 167°C.

Spectre UV (EtOH) PM 406,6

Max. 259 nm $\epsilon$ = 15.700
Max. 300 nm $\epsilon$ = 18.700

Spectre RMN ($CDCl_3$)

2,43 ppm : $CH_3S$
5,76 ppm : $H_4$
4,32 ppm : $H_{11}$
0,55 ppm : $H_{18}$
2,61 et 2,94 ppm : H de l'oxirane
7,02 et 7,13 ppm : aromatiques.

Etude pharmacologique des produits de l'invention.

Etude de l'activité des produits de l'invention sur les récepteurs hormonaux :

Récepteur progestogène de l'utérus de lapine :

Des lapines impubères d'environ 1 kg recoivent une application cutanée de 25 g d'estradiol. 5 jours après ce traitement, les animaux sont sacrifiés, les utérus sont prélevés, pesés et homogénéisés à 0°C, à l'aide d'un Potter teflon-verre dans une solution tamponnée TS (Tris 10mM, saccharose 0,25M, HCl pH 7,4) (1 g de tissu pour 50 ml de TS). L'homogénat est ensuite ultracentrifugé (105 000 g x90 mn) à 0°C. Des aliquotes du surnageant ainsi obtenu sont incubées à 0°C pendant un temps t, avec une concentration constante (T) de produit R tritié (17,21-diméthyl 19-nor 4,9-pregnadièn-3,20-dione) en présence de concentrations croissantes (0 - 2500 . $10^{-9}$M) soit de R froid, soit de progestérone froide, soit du produit froid à tester. La concentration de R tritié lié (B) est ensuite mesurée dans chaque incubat par la technique d'adsorption au charbon dextran.

Récepteur glucocorticoïde du thymus de rat :

Des rats mâles Sprague-Dawley EOPS pesant 160 à 200 g sont surrénalectomisés. 4 à 8 jours après cette ablation, les animaux ont sacrifiés et les thymus sont prélevés et homogénéisés à 0°C dans un tampon Tris 10mM, saccharose 0,25M, dithiothreitol 2mM, HCl pH 7,4 à l'aide d'un Potter polytétrafluroéthylène-verre (1 g de tissu pour 10 ml de TS). L'homogénat est ensuite ultracentrifugé (105 000 g x 90 mn) à 0°C. Des aliquotes du surnageant ainsi obtenu sont incubées à 0°C pendant un temps (t) avec une concentration constante (T) de dexaméthasone tritiée en présence de concentrations croissantes (0 - 2500.$10^{-9}$M) soit de dexaméthasone froide, soit du produit froid à tester. La concentration de la dexaméthasone tritiée liée (B) est ensuite mesurée dans chaque incubat par la technique d'adsorption au charbon-dextran.

Calcul de l'affinité relative de liaison :

Le calcul de l'affinité relative de liaison (ARL) est identique pour tous les récepteurs.
On trace les 2 courbes suivantes : le pourcentage de l'hormone tritiée liée $\frac{B}{T}$ en fonction du logarithme de la concentration de l'hormone de référence froide et $\frac{B}{T}$ en fonction du logarithme de la concentration du produit froid testé.
On détermine la droite d'équation

$$I^{50} = \left(\frac{B}{T}\max + \frac{B}{T}\min\right)/2$$

$\frac{B}{T}$ max = pourcentage de l'hormone tritiée liée pour une incubation de cette hormone tritiée à la concentration (T). $\frac{B}{T}$ min = pourcentage de l'hormone tritiée liée pour une incubation de cette hormone tritiée à la concentration (T) en présence d'un grand excès d'hormone froide (2500.$10^{-9}$M). Les intersections de la droite $I_{50}$ et des courbes permettent d'évaluer les concentrations de l'hormone de référence froide (CH) et du produit froid testé (CX) qui inhibent de 50 % la liaison de l'hormone tritiée sur le récepteur.
L'affinité relative de liaison (ARL) du produit testé est déterminée par l'équation :

$$ARL = 100 \frac{(CH)}{(CX)}$$

Les résultats obtenus sont les suivants :

| Produits des exemples | Temps d'incubation à 0° C | | | |
|---|---|---|---|---|
| | Progestogène | | Glucocorticoïde | |
| | 2 H | 4 H | 2 H | 4 H |
| 1 | 93 | 415 | 126 | 43 |
| 2 | 23 | 67 | 56 | 22 |
| 3 | 24 | 157 | 23 | 24 |

Conclusion :

Les produits étudiés, particulièrement le produit de l'exemple 1 présentent une affinité marquée pour les récepteurs glucocorticoïde et progestogène.

Des résultats obtenus, on peut conclure que les produits peuvent présenter des activités agonistes ou antagonistes des glucocorticoïdes et des progestogènes.

II. Activité antiglucocorticoïde :

La technique utilisée découle de la méthode décrite par Dausse et Coll. dans Molecular Pharmacology 13, 948 - 955 (1977) ("the relationship beetween glucocorticoïd structure and effects upon Thymoxytes"), pour des thymocytes de souris.

Des thymocytes de rats surrénalectomisés sont incubés à 37° C pendant 3 heures, dans un milieu nutritif renfermant $5.10^{-8}$M de dexaméthasone en présence ou non d'un produit à étudier à différentes concentrations. On ajoute l'uridine tritiée et poursuit l'incubation pendant une heure. On refroidit les incubats, les traite avec une solution d'acide trichloroacétique à 5%, les filtre sur papier Whatman GF/A, les lave trois fois à l'aide d'une solution d'acide trichloroacétique à 5%. On détermine la radioactivité retenue par le filtre.

Les glucocorticoïdes et en particulier la dexaméthasone provoquent une diminution de l'incorporation d'uridine tritiée. Les produits des exemples 1 à 4 s'opposent à cet effet.

| Produit de l'exemple | $5.10^{-8}$M Dexaméthasone + le produit à tester à la concentration de | % d'inhibition de l'effet de la Dexaméthasone |
|---|---|---|
| 1 | $10^{-8}$M<br>$10^{-7}$M<br>$10^{-6}$M | 16<br>55<br>122 |
| 2 | $10^{-8}$M<br>$10^{-7}$M<br>$10^{-6}$M | 10<br>27<br>76 |
| 3 | $10^{-8}$M<br>$10^{-7}$M<br>$10^{-6}$M | 7<br>22<br>93 |

Il a par ailleurs été constaté qu'utilisés seuls les produits testés ne provoquent aucun effet du type glucocorticoïde.

Conclusion :

Les produits étudiés présentent une activité anti-glucocorticoïde très marquée tout en étant dépourvus d'activité glucocorticoïde.

III. Activité abortive chez la rate.

On détermine le jour $J_1$ de la gestation par la présence de spermatozoïdes dans le frottis vaginal. Au jour $J_9$ de la gestation, on administre le produit en suspension dans la carboxyméthyl cellulose contenant 0,5% de tween.

Les animaux ont sacrifiés 72 heures après le traitement et l'utérus est examiné pour déterminer l'état de la gestation.

On constate un avortement complet sur tous les animaux du groupe avec le produit de l'exemple 1 administré à la dose de 3 mg/kg.

**Revendications**

**Revendications pour les Etats contractants suivants : AT, BE, CH, DE, FR, GB, IT, LI, LU, NL, SE**

**1.** Les produits de formule générale (I) :

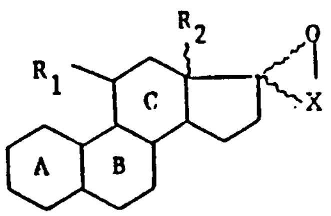

(I)

dans laquelle $R_1$ représente un radical aryle ou aralkyle carbocyclique ou hétérocyclique éventuellement substitué, $R_2$ en position alpha ou béta, représente un radical hydrocarboné renfermant de 1 à 18 atomes de carbone, le trait ondulé du spiro éther indique que l'atome d'oxygène peut se trouver en position alpha ou béta, X représente un radical $-(CH_2)_n-$ dans lequel n représente un entier égal à 1,2 ou 4, ou X représente un radical $-CH=CH-CH_2-CH_2$ les cycles A et B ayant l'une des structures suivantes :

a) - soit A et B représentent le groupement :

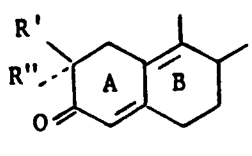

dans lequel R′ et R″ identiques ou différents représentent un atome d'hydrogène, ou un radical alkyle ayant de 1 à 4 atomes de carbone,

b) - soit A et B représentent le groupement :

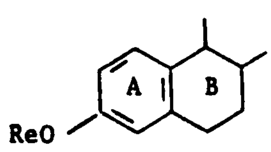

dans lequel Re représente un atome d'hydrogène, un radical alkyle ayant de 1 à 6 atomes de carbone éventuellement substitué ou un radical acyle ;

c) - soit A, B et C représentent le groupement :

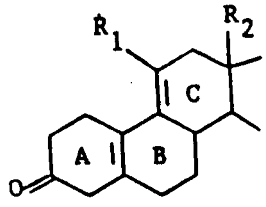

d) - soit A et B représentent le groupement :

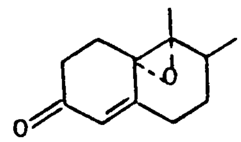

e) - soit A et B représentent le groupement :

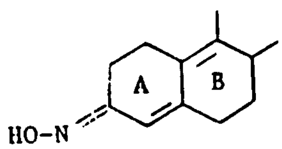

ainsi que leurs sels.

**2.** Les produits de formule (I) telle que définie à la revendication 1, répondant à la formule (I′) :

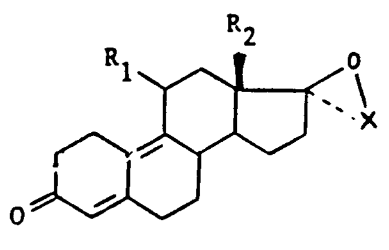

(I')

dans laquelle $R_1$ représente un radical aryle ou aralkyle carbocyclique ou hétérocyclique éventuellement substitué, $R_2$ en position béta, représente un radical hydrocarboné renferment de 1 à 18 atomes de carbone, l'atome d'oxygène du spiro éther est en position 17béta, X représente un radical $-(CH_2)_n$ dans lequel n représente un entier égal à 2 ou 4 ou X représente un radical $-CH=CH-CH_2-CH_2-$ ainsi que leurs sels.

**3.** Les produits de formule (I) telle que définie à l'une quelconque des revendications 1 ou 2, dans laquelle $R_1$ représente soit un radical aryle ou aralkyle portant une fonction amine :

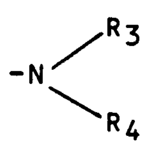

dans laquelle $R_3$ et $R_4$ représentent chacun un radical alkyle primaire, secondaire ou tertiaire

renfermant de 1 à 8 atomes de carbone ou $R_3$ et $R_4$ forment avec l'atome d'azote auquel ils sont liés un hétérocycle comportant éventuellement un autre hétéroatome choisi dans le groupe formé par l'oxygène, l'azote, le soufre et le silicium, soit un radical aryle portant une fonction méthylthio ou éthylthio ainsi que leurs sels.

**4.** Les produits de formule (I) telle que définie à l'une quelconque des revendications 1 à 3, dans laquelle $R_1$ est un radical phényle, le substituant porté par ce radical phényle est en position para, ainsi que leurs sels.

**5.** Les produits de formule (I) telle que définie à l'une quelconque des revendications 1 à 4 dans laquelle $R^1$ représente l'un des radicaux suivants :

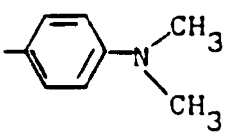
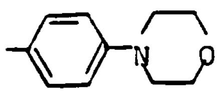
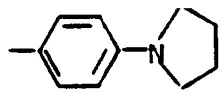
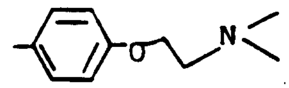
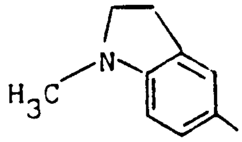
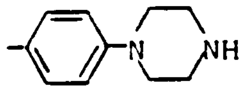
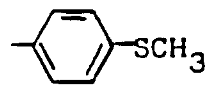
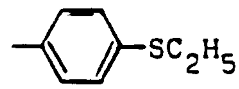
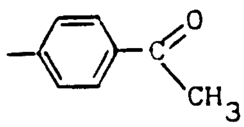 et 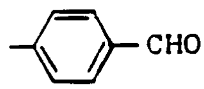

ainsi que leurs sels.

**6.** Les produits de formule (I) telle que définie à la revendication 5, dans laquelle $R_1$ représente un radical :

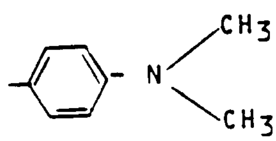

ou un radical :

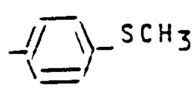

ainsi que leurs sels.

**7.** Les produits de formule (I) telle que définie à l'une quelconque des revendications 1 à 6, dont les noms suivent :

- la (17R) 11béta-/4-(diméthylamino)phényl/spiro (estra-4,9-dièn-17,2'-oxétan)-3-one,
- La (17R) 11béta-/4-(méthylthio)phényl/ spiro (estra-4,9-dièn-17,2'-oxétan)-3-one.

**8.** Procédé de préparation des produits de formule générale (I) telle que définie à la revendication 1 caractérisé en ce que :

a) pour préparer les produits de formule ($I_A$) :

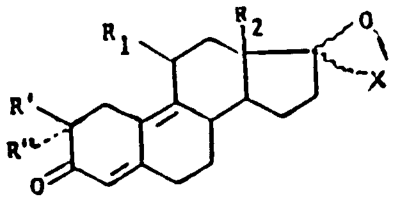

($I_A$)

dans laquelle $R_1$, X et $R_2$ conservent la même signification que dans la revendication 1, R' et R" représentent chacun un atome d'hydrogène ou un radical alkyle ou bien l'un représente un atome d'hydrogène et l'autre représente un radical alkyle, l'on soumet un produit de formule (II) :

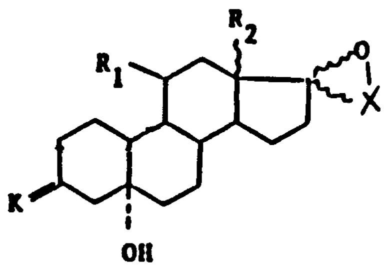

(II)

dans laquelle K représente un groupe cétonique bloqué, à l'action d'un réactif de déshydratation également susceptible de libérer la fonction cétone pour obtenir les produits de formule ($I_A$) dans laquelle R′ et R″ représentent un atome d'hydrogéne, et que, si désiré, l'on soumet à une oxydation les produits de formule ($I_A$) dans laquelle $R_1$ comporte un atome de soufre ou d'azote pour obtenir les produits dans lesquels $R_1$ comporte un atome de soufre oxydé en sulfoxyde ou en sulfone ou un atome d'azote oxydé en N-oxyde, et si désiré, soumet les produits de formule ($I_A$) à l'action d'une base forte puis d'un halogénure d'alkyle pour obtenir un produit de formule ($I_A$) dans laquelle R′ et/ou R″ représentent un radical alkyle ayant de 1 à 4 atomes de carbone ;

b) pour préparer les produits de formule ($I_B$) :

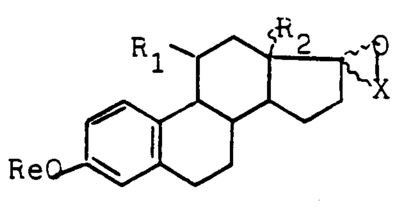

($I_B$)

dans laquelle $R_1$, $R_2$ et Re conservent la même signification qu'à la revendication 1, on soumet un produit de formule ($I'_A$)

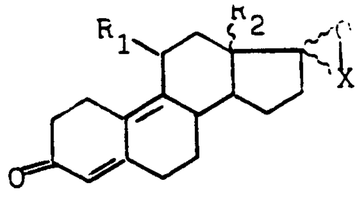

($I'_A$)

dans laquelle $R_1$ et $R_2$ ont la signification déjà indiquée, obtenu comme décrit ci-dessus, à l'action d'un agent d'aromatisation puis le cas échéant à l'action d'un agent de saponification et enfin si désiré soumet le produit de formule ($I_B$) dans laquelle Re est un atome d'hydrogène à un réactif d'alkylation ;

c) pour préparer les produits de formule ($I_C$) :

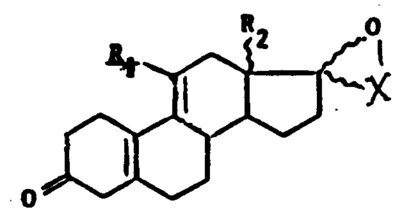

($I_C$)

dans laquelle $R_1$ et $R_2$ conservent la même signification qu'à la revendication 1, on soumet un produit de formule ($I'_A$) à l'action d'un agent d'acylation puis de saponification ;
d) pour préparer les produits de formule ($I_D$) :

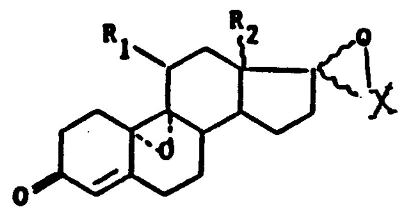

($I_D$)

dans laquelle $R_1$ et $R_2$ conservent la même signification qu'à la revendication 1, on soumet un produit de formule ($I'_A$) à l'action d'un agent d'époxydation ;
e) pour préparer les produits de formule ($I_E$) :

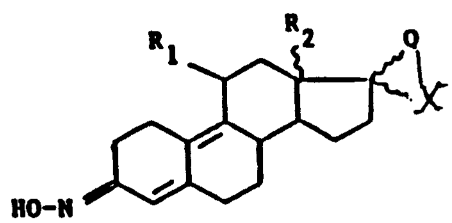

($I_E$)

dans laquelle $R_1$ et $R_2$ conservent la même signification qu'à la revendication 1, on fait agir l'hydroxylamine sur un produit de formule ($I'_A$) ;
f) l'on salifie, le cas échéant, les produits de formule $I_A$, $I_B$, $I_C$, $I_D$ et $I_E$ obtenus.

**9.** Procédé de préparation des produits de formule générale (I) telle que définie à la revendication 1 dans laquelle X représent un groupement :
$-(CH_2)_4-$ ou $-CH=CH-CH_2-CH_2-$ caractérisé en ce que l'on traite un produit de formule (III) :

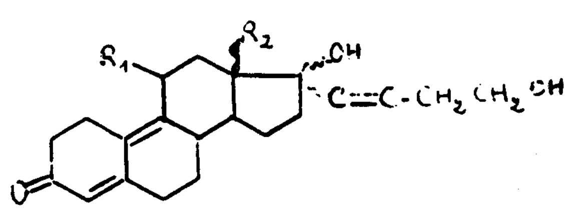

(III)

dans laquelle $R_1$ et $R_2$ conservent la même signification que dans la revendication 1 et le trait pointillé indique la présence éventuelle d'une double liaison entre les carbones qui le portent, à l'action d'un réactif de cyclisation, pour obtenir un produit de formule ($I''_A$) :

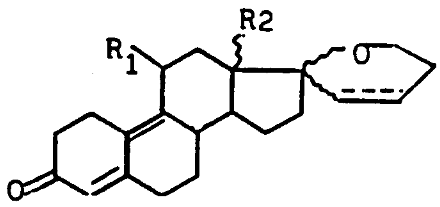

(I"A)

correspondant à un produit de formule ($I_A$) dans laquelle R′ et R″ représentent chacun un atome d'hydrogène et X représente les groupements $-(CH_2)_4$ et $-CH=CH-CH_2-CH_2-$, produits de formule ($I''_A$) que l'on transforme en produits de formule ($I_A$) correspondants dans laquelle l'un des radicaux R′ ou R″ ou les deux radicaux R′ et R″ représentent un radical alkyle ayant de 1 à 4 atomes de carbone, et en produits de formules $I_B$, $I_C$, $I_D$ et $I_E$ selon le procédé décrit à la revendication 8.

**10.** A titre de médicaments, les produits de formule générale (I) telle que définie à la revendication 1, ainsi que leurs sels pharmaceutiquement acceptables.

**11.** A titre de médicaments, les produits de formule générale (I) telle que définie à l'une des revendications 2 à 6 ainsi que leurs sels pharmaceutiquement acceptables.

**12.** A titre de médicaments, les produits de formule générale (I) tels que définis à la revendication 7, ainsi que leurs sels pharmaceutiquement acceptables.

**13.** Compositions pharmaceutiques renfermant comme principe actif, un au moins des médicaments selon l'une quelconque des revendications 10 à 12.

**14.** A titre de produits industriels nouveaux, les produits de formule (II′) :

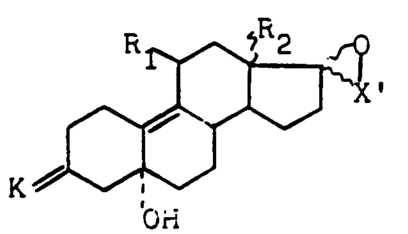

(II')

dans laquelle $R_1$, $R_2$ et K ont la signification indiquée à la revendication 8 et X′ représente $-(CH_2)_{n1}-$ dans lequel $n_1 = 2$ ou 4 ou X′ représente : $-CH=CH-CH_2-CH_2$.

**Revendications pour l'Etat contractant suivant : ES**

**1.** Procédé de préparation, des produits de formule générale (I) :

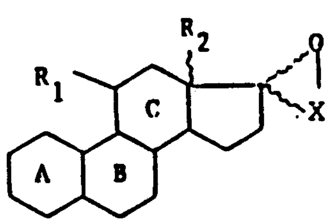

(I)

dans laquelle $R_1$ représente un radical aryle ou aralkyle carbocyclique ou hétérocyclique éventuellement substitué, $R_2$ en position alpha ou béta, représente un radical hydrocarboné renfermant de 1 à 18 atomes de carbone, le trait ondulé du spiro éther indique que l'atome d'oxygène peut se trouver en

position alpha ou béta, X représente un radical $-(CH_2)_n-$ dans lequel n représente un entier égal à 1,2 ou 4, ou X représente un radical $-CH=CH-CH_2-CH_2-$ les cycles A et B ayant l'une des structures suivantes :

a) - soit A et B représentent le groupement :

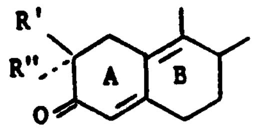

dans lequel R' et R" identiques ou différents représentent un atome d'hydrogène, ou un radical alkyle ayant de 1 à 4 atomes de carbone,

b) - soit A et B représentent le groupement :

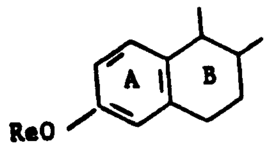

dans lequel Re représente un atome d'hydrogène, un radical alkyle ayant de 1 à 6 atomes de carbone éventuellement substitué ou un radical acyle ;

c) - soit A, B et C représentent le groupement :

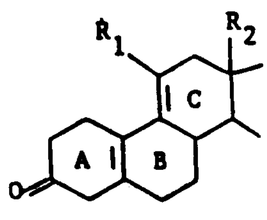

d) - soit A et B représentent le groupement :

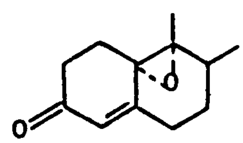

e) - soit A et B représentent le groupement :

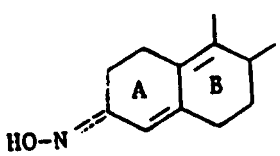

ainsi que de leurs sels, caractérisé en ce que :

a) pour préparer les produits de formule ($I_A$) :

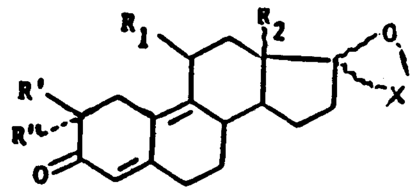

($I_A$)

dans laquelle $R_1$ X et $R_2$ conservent la même signification que précédemment, R′ et R″ représentent chacun un atome d'hydrogène ou un radical alkyle ou bien l'un représente un atome d'hydrogène et l'autre représente un radical alkyle, l'on soumet un produit de formule (II) :

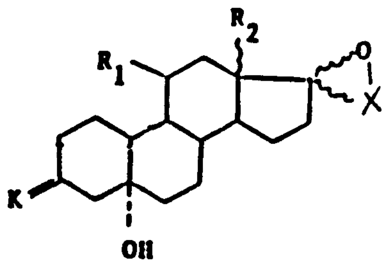

(II)

dans laquelle K représente un groupe cétonique bloqué, à l'action d'un réactif de déshydratation également susceptible de libérer la fonction cétone pour obtenir les produits de formule ($I_A$) dans laquelle R′ et R″ représentent un atome d'hydrogène, et que, si désiré, l'on soumet à une oxydation les produits de formule ($I_A$) dans laquelle $R_1$ comporte un atome de soufre ou d'azote pour obtenir les produits dans lesquels $R_1$ comporte un atome de soufre oxydé en sulfoxyde ou en sulfone ou un atome d'azote oxydé en N-oxyde, et si désiré, soumet les produits de formule ($I_A$) à l'action d'une base forte puis d'un halogénure d'alkyle pour obtenir un produit de formule ($I_A$) dans laquelle R′ et/ou R″ représentent un radical alkyle ayant de 1 à 4 atomes de carbone ;
b) pour préparer les produits de formule ($I_B$) :

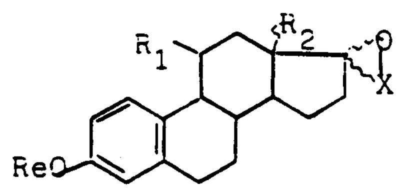

($I_B$)

dans laquelle $R_1$, $R_2$ et Re conservent la même signification que précédemment, on soumet un produit de formule ($I'_A$)

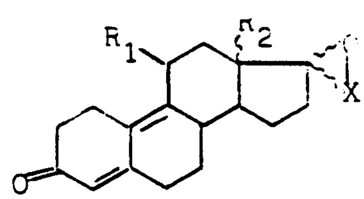

($I'_A$)

dans laquelle $R_1$ et $R_2$ ont la signification déjà indiquée, obtenu comme décrit ci-dessus, à l'action d'un agent d'aromatisation puis le cas échéant à l'action d'un agent de saponification et enfin si désiré sou-met le produit de formule ($I_B$) dans laquelle Re est un atome d'hydrogène à un

réactif d'alkylation ;
c) pour préparer les produits de formule ($I_C$) :

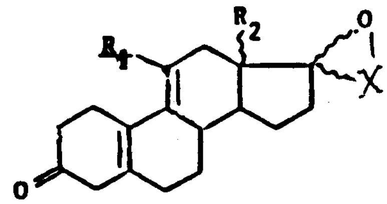

($I_C$)

dans laquelle $R_1$ et $R_2$ conservent la même signification que précédemment, on soumet un produit de formule ($I'_A$) à l'action d'un agent d'acylation puis de saponification ;
d) pour préparer les produits de formule ($I_D$) :

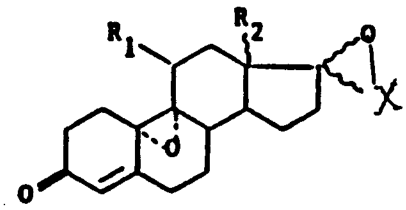

($I_D$)

dans laquelle $R_1$ et $R_2$ conservent la même signification que précédemment, on soumet un produit de formule ($I'_A$) à l'action d'un agent d'époxydation ;
e) pour préparer les produits de formule ($I_E$) :

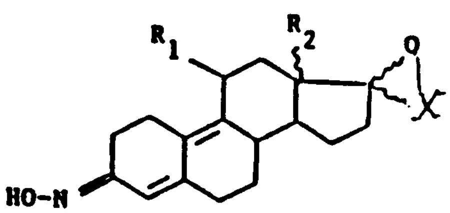

($I_E$)

dans laquelle $R_1$ et $R_2$ conservent la même signification que précédemment, on fait agir l'hydroxylamine ur un produit de formule ($I'_A$) ;
f) l'on salifie, le cas échéant, les produits de formule $I_A$, $I_B$, $I_C$, $I_D$ et $I_E$ obtenus.

**2.** Procédé de préparation des produits de formule générale (I) telle que définie à la revendication 1 dans laquelle X représente un groupement :
$-(CH_2)_4-$ ou $-CH=CH-CH_2-CH_2-$ caractérisé en ce que l'on traite un produit de formule (III) :

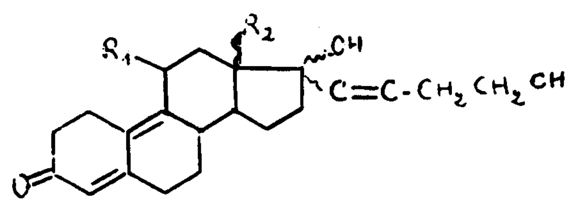

(III)

dans laquelle $R_1$ et $R_2$ conservent la même signification que dans la revendication 1 et le trait pointillé indique la présence éventuelle d'une double liaison entre les carbones qui le portent, à l'action d'un réactif de cyclisation, pour obtenir un produit de formule ($I''_A$) :

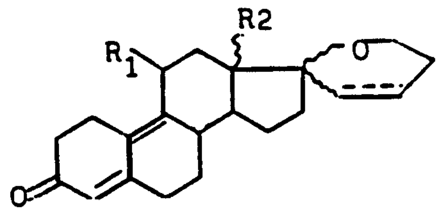

(I"$_A$)

correspondant à un produit de formule ($I_A$) dans laquelle R′ et R″ représentent chacun un atome d'hydrogène et X représente les groupements $-(CH_2)_4$ et $-CH=CH-CH_2-CH_2-$, produits de formule ($I''_A$) que l'on transforme en produits de formule ($I_A$) correspondants dans laquelle l'un des radicaux R′ ou R″ ou les deux radicaux R′ et R″ représentent un radical alkyle ayant de 1 à 4 atomes de carbone, et en produits de formules $I_B$, $I_C$, $I_D$ et $I_E$ selon le procédé décrit à la revendication 1.

**3.** Procédé selon la revendication 1, caractérisé en ce que l'on utilise au départ un produit de formule (II) dans laquelle $R_1$ représente un radical aryle ou aralkyle carbocyclique ou hétérocyclique éventuellement substitué, $R_2$ en position béta, représente un radical hydrocarboné renfermant de 1 à 18 atomes de carbone, l'atome d'oxygène du spiro éther est en position 17béta, X représente un radical $-(CH_2)_n$ dans lequel n représente un entier égal à 2 ou 4 ou X représente un radical $-CH=CH-CH_2-CH_2-$.

**4.** Procédé selon la revendication 2, caractérisé en ce que l'on utilise au départ un produit de formule (III) dans laquelle $R_1$ représente un radical aryle ou aralkyle carbocyclique ou hétérocyclique éventuellement substitué, $R_2$ en position béta, représente un radical hydrocarboné renfermant de 1 à 18 atomes de carbone, l'atome d'oxygène du spiro éther est en position 17béta.

**5.** Procédé selon l'une quelconque des revendications 1 à 4, caractérisé en ce que l'on utilise au départ un produit de formule (II) ou (III) dans laquelle $R_1$ représente soit un radical aryle ou aralkyle portant une fonction amine :

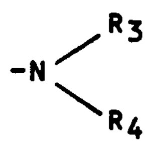

dans laquelle $R_3$ et $R_4$ représentent chacun un radical alkyle primaire, secondaire ou tertiaire renfermant de 1 à 8 atomes de carbone ou $R_3$ et $R_4$ forment avec l'atome d'azote auquel ils sont liés un hétérocycle comportant éventuellement un autre hétéroatome choisi dans le groupe formé par l'oxygène, l'azote, le soufre et le silicium, soit un radical aryle portant une fonction méthylthio ou éthylthio.

**6.** Procédé elon l'une quelconque des revendications 1 à 5, caractérisé en ce que l'on utilise au départ un produit de formule (II) ou (III) dans laquelle $R_1$ est un radical phényle, le substituant porté par ce radical phényle est en position para.

**7.** Procédé selon l'une quelconque des revendications 1 à 6, caractérisé en ce que l'on utilise au départ un produit de formule (II) ou (III) dans laquelle $R_1$ représente l'un des radicaux suivants :

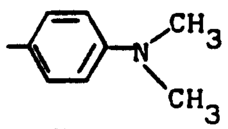
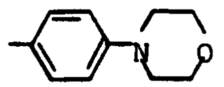
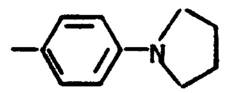
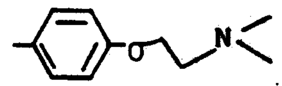

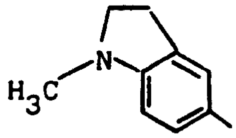
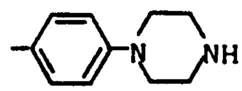
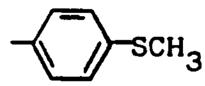
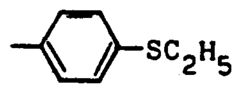

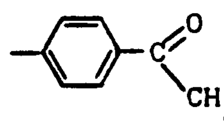 et 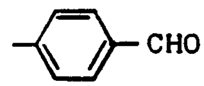

**8.** Procédé selon l'une quelconque des revendications 1 à 7, caractérisé en ce que l'on utilise au départ un produit de formule (II) ou (III) dans laquelle $R_1$ représente un radical :

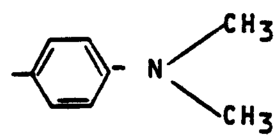

ou un radical :

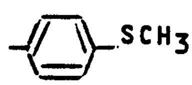

**9.** Procédé selon la revendication 8, caractérisé en ce que l'on choisit le produit de formule (II) ou (III) de manière telle que l'on prépare :

- la (17R) 11béta-/4-(diméthylamino)phényl/spiro (estra-4,9-dièn-17,2'-oxétan)-3-one,
- la (17R) 11 béta-/4-(méthylthio)phényl/ spiro (estra-4,9-dièn-17,2'-oxétan)-3-one.

**Revendications pour l'Etat contractant suivant : GR**

**1.** Procédé de préparation, des produits de formule générale (I) :

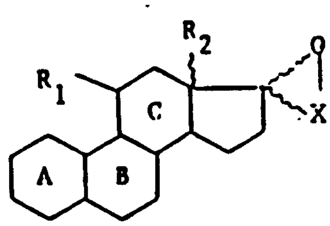

(I)

dans laquelle $R_1$ représente un radical aryle ou aralkyle carbocyclique ou hétérocyclique éventuellement substitué, $R_2$ en position alpha ou béta, représente un radical hydrocarboné renfermant de 1 à 18 atomes de carbone, le trait ondulé du spiro éther indique que l'atome d'oxygène peut se trouver en position alpha ou béta, X représente un radical $-(CH_2)_n-$ dans lequel n représente un entier égal à 1,2 ou 4, ou X représente un radical $-CH=CH-CH_2-CH_2-$ les cycles A et B ayant l'une des structures suivantes :

a) - soit A et B représentent le groupement :

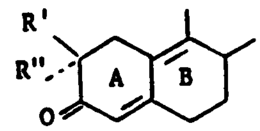

dans lequel R' et R" identiques ou différents représentent un atome d'hydrogène, ou un radical alkyle ayant de 1 à 4 atomes de carbone,
b) - soit A et B représentent le groupement :

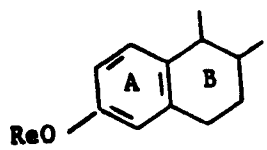

dans lequel Re représente un atome d'hydrogène, un radical alkyle ayant de 1 à 6 atomes de carbone éventuellement substitué ou un radical acyle ;
c) - soit A, B et C représentent le groupement :

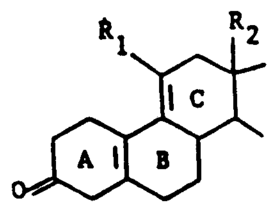

d) - soit A et B représentent le groupement :

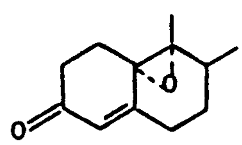

e) - soit A et B représentent le groupement :

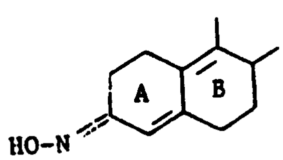

ainsi que de leurs sels, caractérisé en ce que :

a) pour préparer les produits de formule ($I_A$) :

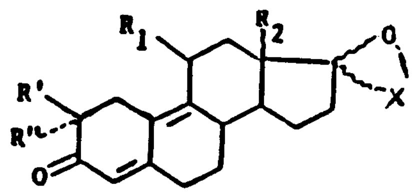

($I_A$)

dans laquelle $R_1$ X et $R_2$ conservent la même signification que précédemment, $R'$ et $R''$ représentent chacun un atome d'hydrogène ou un radical alkyle ou bien l'un représente un atome d'hydrogène et l'autre représente un radical alkyle, l'on soumet un produit de formule (II) :

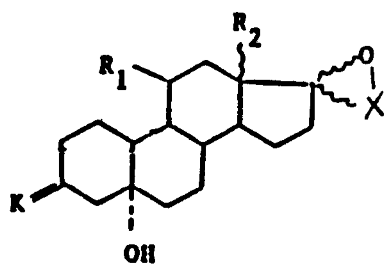

(II)

dans laquelle K représente un groupe cétonique bloqué, à l'action d'un réactif de déshydratation également susceptible de libérer la fonction cétone pour obtenir les produits de formule ($I_A$) dans laquelle $R'$ et $R''$ représentent un atome d'hydrogène, et que, si désiré, l'on soumet à une oxydation les produits de formule ($I_A$) dans laquelle $R_1$ comporte un atome de soufre ou d'azote pour obtenir les produits dans lesquels $R_1$ comporte un atome de soufre oxydé en sulfoxyde ou en sulfone ou un atome d'azote oxydé en N-oxyde, et si désiré, soumet les produits de formule ($I_A$) à l'action d'une base forte puis d'un halogénure d'alkyle pour obtenir un produit de formule ($I_A$) dans laquelle $R'$ et/ou $R''$ représentent un radical alkyle ayant de 1 à 4 atomes de carbone ;
b) pour préparer les produits de formule ($I_B$) :

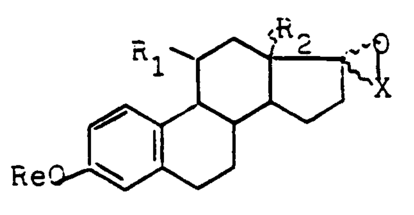

($I_B$)

dans laquelle $R_1$, $R_2$ et Re conservent la même signification que précédemment, on soumet un produit de formule ($I'_A$)

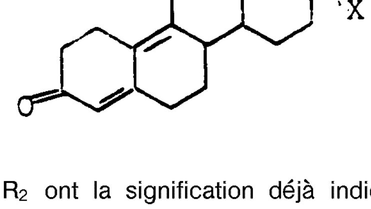

($I'_A$)

dans laquelle $R_1$ et $R_2$ ont la signification déjà indiquée, obtenu comme décrit ci-dessus, à l'action d'un agent d'aromatisation puis le cas échéant à l'action d'un agent de saponification et enfin si désiré sou-met le produit de formule ($I_B$) dans laquelle Re est un atome d'hydrogène à un

réactif d'alkylation ;
c) pour préparer les produits de formule ($I_C$) :

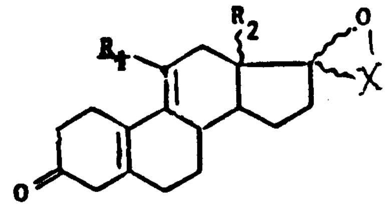

($I_C$)

dans laquelle $R_1$ et $R_2$ conservent la même signification que précédemment, on soumet un produit de formule ($I'_A$) à l'action d'un agent d'acylation puis de saponification ;
d) pour préparer les produits de formule ($I_D$) :

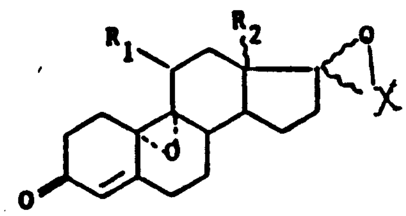

($I_D$)

dans laquelle $R_1$ et $R_2$ conservent la même signification que précédemment, on soumet un produit de formule ($I'_A$) à l'action d'un agent d'époxydation ;
e) pour préparer les produits de formule ($I_E$) :

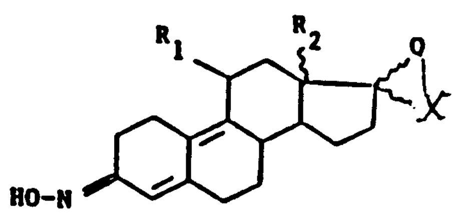

($I_E$)

dans laquelle $R_1$ et $R_2$ conservent la même signification que précédemment, on fait agir l'hydroxylamine sur un produit de formule ($I'_A$) ;
f) l'on salifie, le cas échéant, les produits de formule $I_A$, $I_B$, $I_C$, $I_D$ et $I_E$ obtenus.

**2.** Procédé de préparation des produits de formule générale (I) telle que définie à la revendication 1 dans laquelle X représente un groupement :
$-(CH_2)_4-$ ou $-CH=CH-CH_2-CH_2-$ caractérisé en ce que l'on traite un produit de formule (III) :

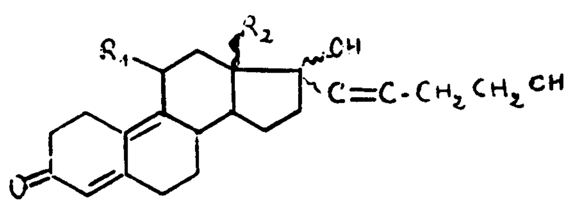

(III)

dans laquelle $R_1$ et $R_2$ conservent la même signification que dans la revendication 1 et le trait pointillé indique la présence éventuelle d'une double liaison entre les carbones qui le portent, à l'action d'un réactif de cyclisation, pour obtenir un produit de formule ($I''_A$) :

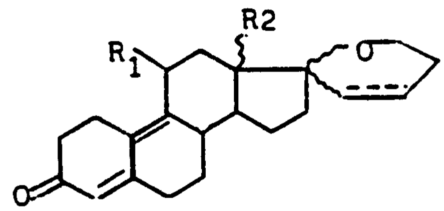

(I"A)

correspondant à un produit de formule ($I_A$) dans laquelle R′ et R″ représentent chacun un atome d'hydrogène et X représente les groupements $-(CH_2)_4$ et $-CH=CH-CH_2-CH_2-$, produits de formule ($I''_A$) que l'on transforme en produits de formule ($I_A$) correspondants dans laquelle l'un des radicaux R′ ou R″ou les deux radicaux R′ et R″ représentent un radical alkyle ayant de 1 à 4 atomes de carbone, et en produits de formules $I_B$, $I_C$, $I_D$ et $I_E$ selon le procédé décrit à la revendication 1.

**3.** Procédé selon la revendication 1, caractérisé en ce que l'on utilise au départ un produit de formule (II) dans laquelle $R_1$ représente un radical aryle ou aralkyle carbocyclique ou hétérocyclique éventuellement substitué, $R_2$ en position béta, représente un radical hydrocarboné renfermant de 1 à 18 atomes de carbone, l'atome d'oxygène du spiro éther est en position 17béta, X représente un radical $-(CH_2)_n$ dans lequel n représente un entier égal à 2 ou 4 ou X représente un radical $-CH=CH-CH_2-CH_2-$.

**4.** Procédé selon la revendication 2, caractérisé en ce que l'on utilise au départ un produit de formule (III) dans laquelle $R_1$ représente un radical aryle ou aralkyle carbocyclique ou hétérocyclique éventuellement substitué, $R_2$ en position béta, représente un radical hydrocarboné renfermant de 1 à 18 atomes de carbone, l'atome d'oxygène du spiro éther est en position 17béta.

**5.** Procédé selon l'une quelconque des revendications 1 à 4, caractérisé en ce que l'on utilise au départ un produit de formule (II) ou (III) dans laquelle $R_1$ représente soit un radical aryle ou aralkyle portant une fonction amine :

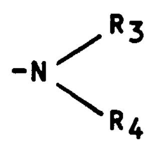

dans laquelle $R_3$ et $R_4$ représentent chacun un radical alkyle primaire, secondaire ou tertiaire renfermant de 1 à 8 atomes de carbone ou $R_3$ et $R_4$ forment avec l'atome d'azote auquel ils sont liés un hétérocycle comportant éventuellement un autre hétéroatome choisi dans le groupe formé par l'oxygène, l'azote, le soufre et le silicium, soit un radical aryle portant une fonction méthylthio ou éthylthio.

**6.** Procédé selon l'une quelconque des revendications 1 à 5, caractérisé en ce que l'on utilise au départ un produit de formule (II) ou (III) dans laquelle $R_1$ est un radical phényle, le substituant porté par ce radical phényle est en position para.

**7.** Procédé selon l'une quelconque des revendications 1 à 6, caractérisé en ce que l'on utilise au départ un produit de formule (II) ou (III) dans laquelle $R_1$ représente l'un des radicaux suivants :

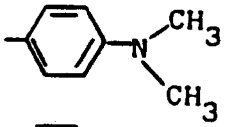
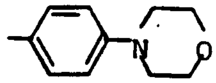
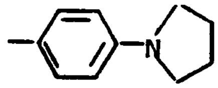
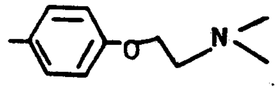
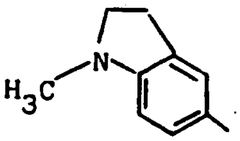
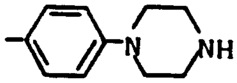
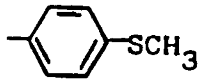
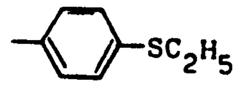
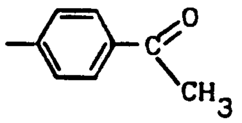
et
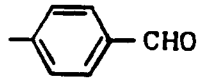

8. Procédé selon l'une quelconque des revendications 1 à 7, caractérisé en ce que l'on utilise au départ un produit de formule (II) ou (III) dans laquelle $R_1$ représente un radical :

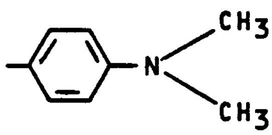

ou un radical :

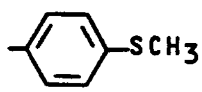

9. Procédé selon la revendication 8, caractérisé en ce que l'on choisit le produit de formule (II) ou (III) de manière telle que l'on prépare :
   - la (17R) 11béta-/4-(diméthylamino)phényl/spiro (estra-4,9-dièn-17,2'-oxétan)-3-one,
   - la (17R) 11béta-/4-(méthylthio)phényl/ spiro (estra-4,9-dièn-17,2'-oxétan)-3-one.

10. Procédé de préparation de compositions pharmaceutiques, caractérisé en ce que l'on met, à titre de principe actif, l'un au moins des produits de formule (I), tels que définis à la revendication 1, ou l'un au moins de leurs sels pharmaceutiques acceptables, sous une forme destinée à cet usage.

11. Procédé de préparation de compositions pharmaceutiques, caractérisé en ce que l'on met, à titre de principe actif, l'un au moins des produits de formule (I), tels que définis à l'une quelconque des revendications 3 à 8, ou l'un au moins de leurs sels pharmaceutiques acceptables, sous une forme destinée à cet usage.

12. Procédé de préparation de compositions pharmaceutiques, caractérisé en ce que l'on met, à titre de principe actif, l'un au moins des produits de formule (I), tels que définis à la revendication 9, ou l'un au moins de leurs sels pharmaceutiques acceptables, sous une forme destinée à cet usage.

**13.** A titre de produits industriels nouveaux, les produits de formule (II') :

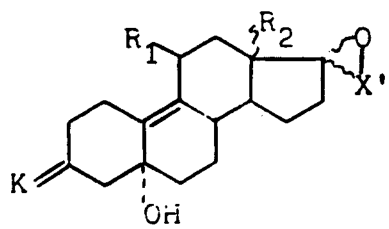

(II')

dans laquelle $R_1$, $R_2$ et K ont la signification indiquée à la revendication 8 et X' représente $-(CH_2)_{n1}-$ dans lequel $n_1$ = 2 ou 4 ou X' représente : $-CH=CH-CH_2-CH_2$.

**Claims**

**Claims for the following Contracting States : AT, BE, CH, DE, FR, GB, IT, LI, LU, NL, SE**

**1.** The products of general formula (I):

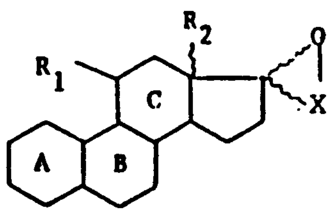

(I)

in which $R_1$ represents an optionally substituted carbocyclic or heterocyclic aryl or aralkyl radical, $R_2$ in the alpha or beta position represents a hydrocarbon radical containing 1 to 18 carbon atoms, the wavy line of the spiro ether indicates that the oxygen atom can be found in the alpha or beta position, X represents a $-(CH_2)_n-$ radical in which n represents an integer equal to 1, 2 or 4, or X represents a $-CH=CH-CH_2-CH_2-$ radical, the rings A and B having one of the following structures:

a) - either A and B represent the group:

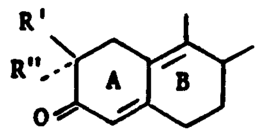

in which R' and R", identical or different, represent a hydrogen atom, or an alkyl radical having 1 to 4 carbon atoms,

b) - or A and B represent the group:

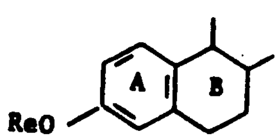

in which Re represents a hydrogen atom, an optionally substituted alkyl radical having 1 to 6 carbon atoms or an acyl radical,

c) - or A, B and C represent the group:

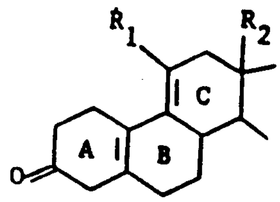

d) - or A and B represent the group:

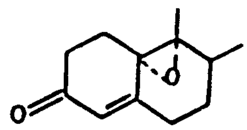

e) - or A and B represent the group:

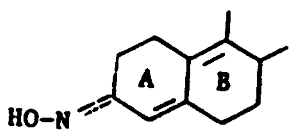

as well as their salts.

**2.** The products of formula (I) as defined in claim 1, corresponding to formula (I'):

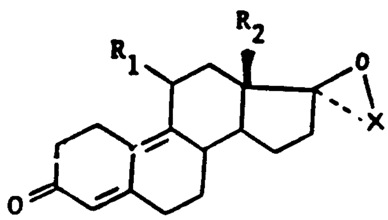

(I')

in which $R_1$ represents an optionally substituted carbocyclic or heterocyclic aryl or aralkyl radical, $R_2$ in the beta position represents a hydrocarbon radical containing 1 to 18 carbon atoms, the oxygen atom of the spiro ether is in the 17beta position, X represents a $-(CH_2)_n$ radical in which n represents an integer equal to 2 or 4 or X represents a $-CH=CH-CH_2-CH_2-$ radical, as well as their salts.

**3.** The products of formula (I) as defined in any one of claims 1 or 2, in which $R_1$ represents either an aryl or aralkyl radical carrying an amine function:

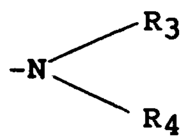

in which $R_3$ and $R_4$ each represent a primary, secondary or tertiary alkyl radical containing 1 to 8 carbon atoms or $R_3$ and $R_4$ form with the nitrogen atom to which they are linked a heterocycle optionally containing another heteroatom chosen from the group formed by oxygen, nitrogen, sulphur

and silicon, or an aryl radical carrying a methylthio or ethylthio function, as well as their salts.

**4.** The products of formula (I) as defined in any one of claims 1 to 3, in which $R_1$ is a phenyl radical, the substituent carried by this phenyl radical is in the para position, as well as their salts.

**5.** The products of formula (I) as defined in any one of claims 1 to 4 in which $R_1$ represents one of the following radicals:

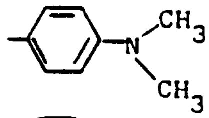
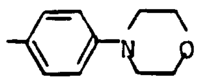
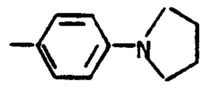
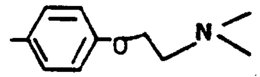
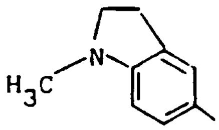
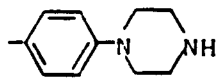
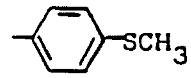
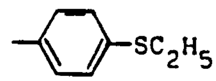
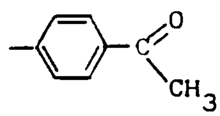

and

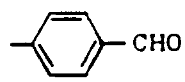

as well as their salts.

**6.** The products of formula (I) as defined in claim 5, in which $R_1$ represents a radical:

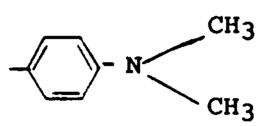

or a radical:

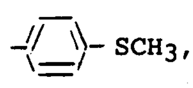

as well as their salts.

**7.** The products of formula (I) as defined in any one of claims 1 to 6, of which the names follow:
- (17R) 11beta-[4-(dimethylamino)phenyl] spiro (estra-4,9-dien-17,2'-oxetan)-3-one,
- (17R) 11beta-[4-(methylthio)phenyl] spiro (estra-4,9-dien-17,2'-oxetan)-3-one.

**8.** Preparation process for the products of general formula (I) as defined in claim 1, characterized in that:

a) to prepare the products of formula ($I_A$):

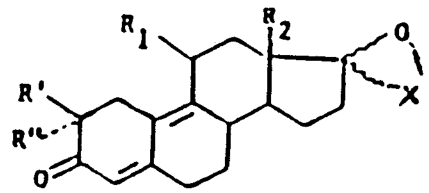

($I_A$)

in which $R_1$, X and $R_2$ keep the same meaning as in claim 1, R' and R" each represent a hydrogen atom or an alkyl radical or one represents a hydrogen atom and the other represents an alkyl radical, a product of formula (II):

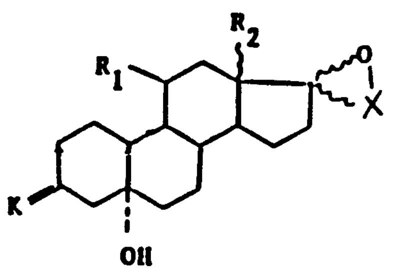

(II)

in which K represents a blocked ketone group, is subjected to the action of a dehydration reagent also capable of releasing the ketone function, in order to obtain the products of formula ($I_A$) in which R' and R" represent a hydrogen atom, and in that, if desired, the products of formula ($I_A$) in which $R_1$ contains a sulphur or nitrogen atom are subjected to an oxidation in order to obtain the products in which $R_1$ contains a sulphur atom oxidized into a sulphoxide or into a sulphone or a nitrogen atom oxidized into an N-oxide, and if desired, the products of formula ($I_A$) are subjected to the action of a strong base then of an alkyl halide in order to obtain a product of formula ($I_A$) in which R' and/or R" represent an alkyl radical having 1 to 4 carbon atoms;

b) to prepare the products of formula ($I_B$):

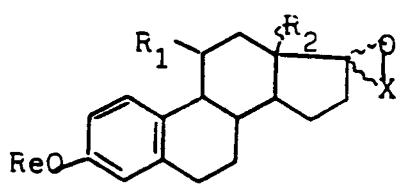

($I_B$)

in which $R_1$, $R_2$ and Re keep the same meaning as in claim 1, a product of formula ($I'_A$):

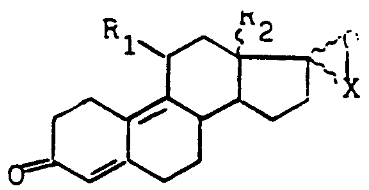

($I'_A$)

in which $R_1$ and $R_2$ have the meaning already indicated, obtained as described above, is subjected to the action of an aromatization agent then if appropriate to the action of a saponification agent and finally if desired the product of formula ($I_B$) in which Re is a hydrogen atom is subjected to an alkylation reagent;

c) to prepare the products of formula ($I_C$):

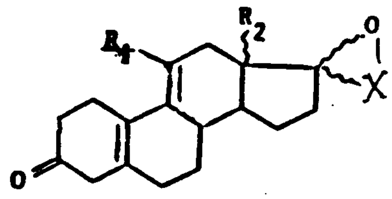

($I_C$)

in which $R_1$ and $R_2$ keep the same meaning as in claim 1, a product of formula ($I'_A$) is subjected to the action of an acylation agent then of a saponification agent;

d) to prepare the products of formula ($I_D$):

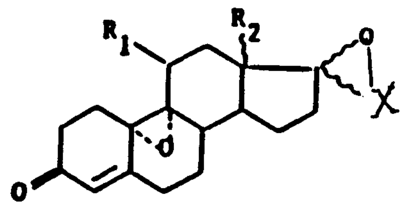

($I_D$)

in which $R_1$ and $R_2$ keep the same meaning as in claim 1, a product of formula ($I'_A$) is subjected to the action of an epoxidation agent;

e) to prepare the products of formula ($I_E$):

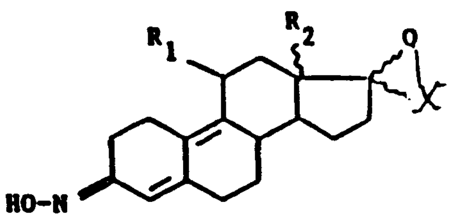

($I_E$)

in which $R_1$ and $R_2$ keep the same meaning as in claim 1, hydroxylamine is reacted on a product of formula ($I'_A$);

f) if appropriate, the products of formulae ($I_A$), ($I_B$), ($I_C$), ($I_D$) and ($I_E$) obtained are salified.

**9.** Preparation process for the products of general formula (I) as defined in claim 1 in which X represents a group:
$-(CH_2)_4-$ or $-CH=CH-CH_2-CH_2-$ characterized in that a product of formula (III):

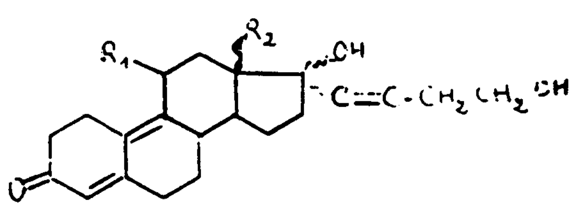

(III)

in which $R_1$ and $R_2$ keep the same meaning as in claim 1 and the wavy line indicates the optional presence of a double bond between the carbons which carry it, is subjected to the action of a cyclization reagent, in order to obtain a product of formula ($I''_A$):

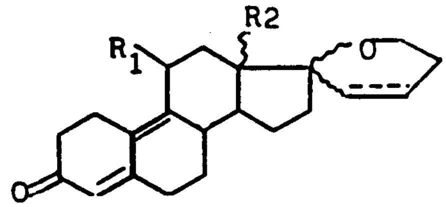

(I"$_A$)

corresponding to a product of formula ($I_A$) in which R' and R" each represent a hydrogen atom and X represents the groups $-(CH_2)_4$ and $-CH=CH-CH_2-CH_2-$, which products of formula (I"$_A$) are converted into corresponding products of formula ($I_A$) in which one of the radicals R' or R" or both radicals R' and R" represent an alkyl radical having 1 to 4 carbon atoms, and into products of formulae $I_B$, $I_C$, $I_D$ and $I_E$ according to the process described in claim 8.

**10.** As medicaments, the products of general formula (I) as defined in claim 1, as well as their pharmaceutically acceptable salts.

**11.** As medicaments, the products of general formula (I) as defined in one of claims 2 to 6, as well as their pharmaceutically acceptable salts.

**12.** As medicaments, the products of general formula (I) as defined in claim 7, as well as their pharmaceutically acceptable salts.

**13.** Pharmaceutical compositions containing as active ingredient at least one of the medicaments according to any one of claims 10 to 12.

**14.** As new industrial products, the products of formula (II'):

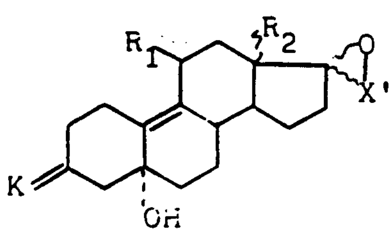

(II')

in which $R_1$, $R_2$ and K have the meaning indicated in claim 8 and X' represents $-(CH_2)_{n1}-$ in which n1 = 2 or 4 or X' represents: $-CH=CH-CH_2-CH_2$.

**Claims for the following Contracting State : ES**

**1.** Preparation process for the products of general formula (I):

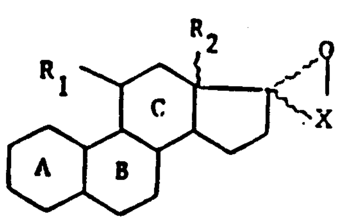

(I)

in which $R_1$ represents an optionally substituted carbocyclic or heterocyclic aryl or aralkyl radical, $R_2$ in

the alpha or beta position represents a hydrocarbon radical containing 1 to 18 carbon atoms, the wavy line of the spiro ether indicates that the oxygen atom can found be in the alpha or beta position, X represents a $-(CH_2)_n-$ radical in which n represents an integer equal to 1, 2 or 4, or X represents a $-CH=CH-CH_2-CH_2-$ radical, the rings A and B having one of the following structures:

a) - either A and B represent the group:

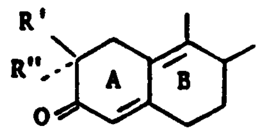

in which R' and R", identical or different, represent a hydrogen atom, or an alkyl radical having 1 to 4 carbon atoms,

b) - or A and B represent the group:

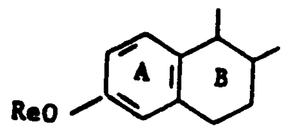

in which Re represents a hydrogen atom, an optionally substituted alkyl radical having 1 to 6 carbon atoms or an acyl radical,

c) - or A, B and C represent the group:

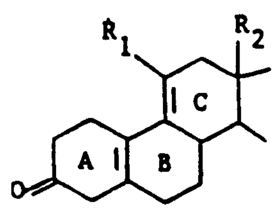

d) - or A and B represent the group:

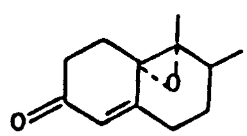

e) - or A and B represent the group:

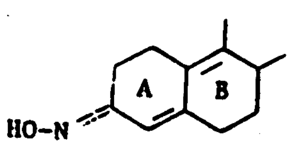

as well as their salts, characterized in that:

a) to prepare the products of formula ($I_A$):

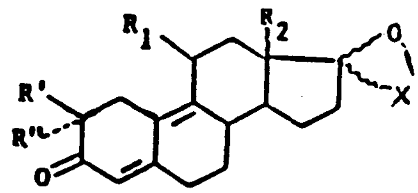

($I_A$)

in which $R_1$, X and $R_2$ keep the same meaning as in claim 1, R' and R" each represent a hydrogen atom or an alkyl radical or one represents a hydrogen atom and the other represents an alkyl radical, a product of formula (II):

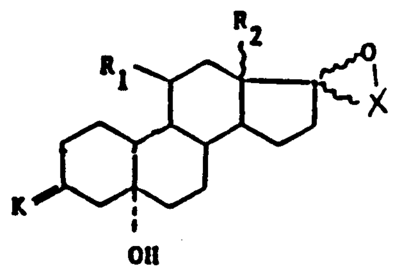

(II)

in which K represents a blocked ketone group, is subjected to the action of a dehydration reagent also capable of releasing the ketone function, in order to obtain the products of formula ($I_A$) in which R' and R" represent a hydrogen atom, and in that, if desired, the products of formula ($I_A$) in which $R_1$ contains a sulphur or nitrogen atom are subjected to an oxidation in order to obtain the products in which $R_1$ contains a sulphur atom oxidized into a sulphoxide or into a sulphone or a nitrogen atom oxidized into an N-oxide, and if desired, the products of formula ($I_A$) are subjected to the action of a strong base then of an alkyl halide in order to obtain a product of formula ($I_A$) in which R' and/or R" represent an alkyl radical having 1 to 4 carbon atoms;
b) to prepare the products of formula ($I_B$):

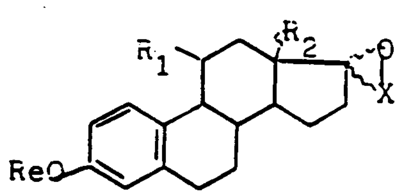

($I_B$)

in which $R_1$, $R_2$ and Re keep the same meaning as in claim 1, a product of formula ($I'_A$):

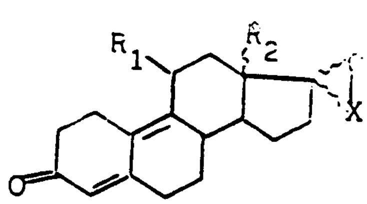

($I'_A$)

in which $R_1$ and $R_2$ have the meaning already indicated, obtained as described above, is subjected to the action of an aromatization agent then if appropriate to the action of a saponification agent and finally if desired the product of formula ($I_B$) in which Re is a hydrogen

atom is subjected to an alkylation reagent;
c) to prepare the products of formula ($I_C$):

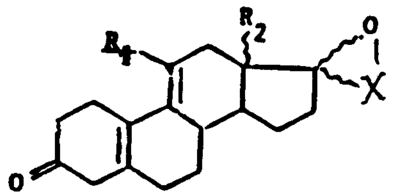

($I_C$)

in which $R_1$ and $R_2$ keep the same meaning as in claim 1, a product of formula ($I'_A$) is subjected to the action of an acylation agent then of a saponification agent;
d) to prepare the products of formula ($I_D$):

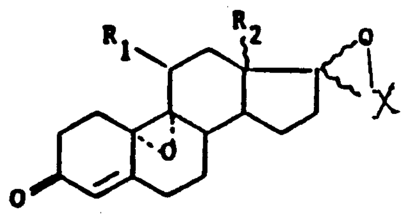

($I_D$)

in which $R_1$ and $R_2$ keep the same meaning as in claim 1, a product of formula ($I'_A$) is subjected to the action of an epoxidation agent;
e) to prepare the products of formula ($I_E$):

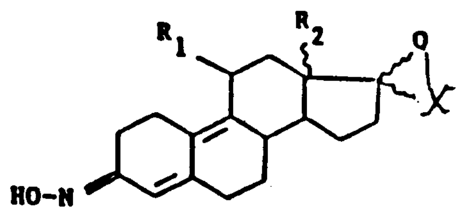

($I_E$)

in which $R_1$ and $R_2$ keep the same meaning as in claim 1, hydroxylamine is reacted on a product of formula ($I'_A$);
f) if appropriate, the products of formulae ($I_A$), ($I_B$), ($I_C$), ($I_D$) and ($I_E$) obtained are salified.

**2.** Preparation process for the products of general formula (I) as defined in claim 1, in which X represents a group:
$-(CH_2)_4-$ or $-CH=CH-CH_2-CH_2-$ characterized in that a product of formula (III):

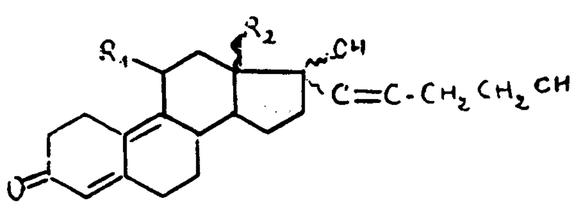

(III)

in which $R_1$ and $R_2$ keep the same meaning as in claim 1 and the wavy line indicates the optional presence of a double bond between the carbons which carry it, is subjected to the action of a cyclization reagent, in order to obtain a product of formula ($I''_A$):

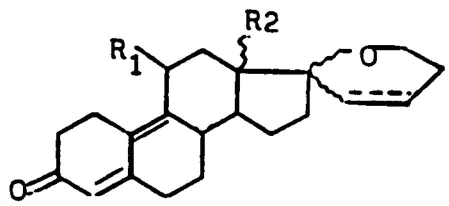

(I"A)

corresponding to a product of formula ($I_A$) in which R' and R" each represent a hydrogen atom and X represents the groups $-(CH_2)_4$ and $-CH=CH-CH_2-CH_2-$, which products of formula ($I"_A$) are converted into corresponding products of formula ($I_A$) in which one of the radicals R' or R" or both radicals R' and R" represent an alkyl radical having 1 to 4 carbon atoms, and into products of formulae $I_B$, $I_C$, $I_D$ and $I_E$ according to the process described in claim 1.

3. Process according to claim 1, characterized in that a product of formula (II) is used at the start in which $R_1$ represents an optionally substituted carbocyclic or heterocyclic aryl or aralkyl radical, $R_2$ in the beta position represents a hydrocarbon radical containing 1 to 18 carbon atoms, the oxygen atom of the spiro ether is in the 17beta position, X represents a $-(CH_2)_n$ radical in which n represents an integer equal to 2 or 4 or X represents a $-CH=CH-CH_2-CH_2-$ radical.

4. Process according to claim 2, characterized in that a product of formula (III) is used at the start in which $R_1$ represents an optionally substituted carbocyclic or heterocyclic aryl or aralkyl radical, $R_2$ in the beta position represents a hydrocarbon radical containing 1 to 18 carbon atoms, the oxygen atom of the spiro ether is in the 17beta position.

5. Process according to any one of claims 1 to 4, characterized in that a product of formula (II) or (III) is used at the start in which $R_1$ represents either an aryl or aralkyl radical carrying an amine function:

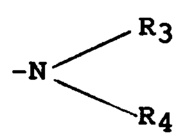

in which $R_3$ and $R_4$ each represent a primary, secondary or tertiary alkyl radical containing 1 to 8 carbon atoms or $R_3$ and $R_4$ form with the nitrogen atom to which they are linked a heterocycle optionally containing another heteroatom chosen from the group formed by oxygen, nitrogen, sulphur and silicon, or an aryl radical carrying a methylthio or ethylthio function.

6. Process according to any one of claims 1 to 5, characterized in that a product of formula (II) or (III) is used at the start in which $R_1$ is a phenyl radical, the substituent carried by this phenyl radical is in the para position.

7. Process according to any one of claims 1 to 6, characterized in that a product of formula (II) or (III) is used at the start in which $R_1$ represents one of the following radicals:

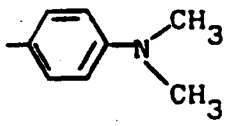 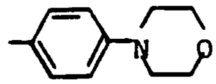 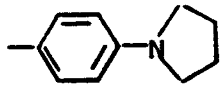 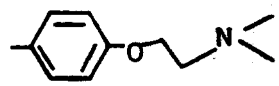

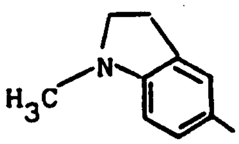 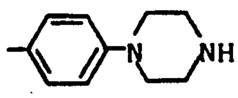 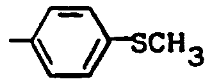 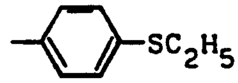

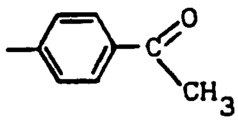 and 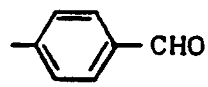

**8.** Process according to any one of claims 1 to 7, characterized in that a product of formula (II) or (III) is used at the start in which $R_1$ represents a radical:

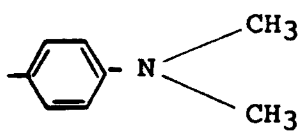

or a radical:

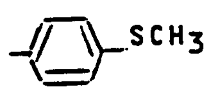

**9.** Process according to claim 8, characterized in that the product of formula (II) or (III) is chosen in such a way that the following are prepared:

- (17R) 11beta-[4-(dimethylamino)phenyl] spiro (estra-4,9-dien-17,2'-oxetan)-3-one,
- (17R) 11beta-[4-(methylthio)phenyl] spiro (estra-4,9-dien-17,2'-oxetan)-3-one.

**Claims for the following Contracting State : GR**

**1.** Preparation process for the products of general formula (I):

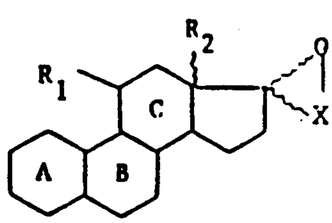

**(I)**

in which $R_1$ represents an optionally substituted carbocyclic or heterocyclic aryl or aralkyl radical, $R_2$ in the alpha or beta position represents a hydrocarbon radical containing 1 to 18 carbon atoms, the wavy line of the spiro ether indicates that the oxygen atom can be found in the alpha or beta position, X represents a $-(CH_2)_n-$ radical in which n represents an integer equal to 1, 2 or 4, or X represents a $-CH=CH-CH_2-CH_2-$ radical, the rings A and B having one of the following structures:

a) - either A and B represent the group:

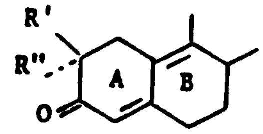

in which R' and R", identical or different, represent a hydrogen atom, or an alkyl radical having 1 to 4 carbon atoms,
b) - or A and B represent the group:

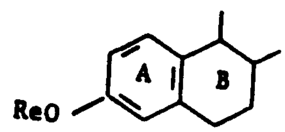

in which Re represents a hydrogen atom, an optionally substituted alkyl radical having 1 to 6 carbon atoms or an acyl radical,
c) - or A, B and C represent the group:

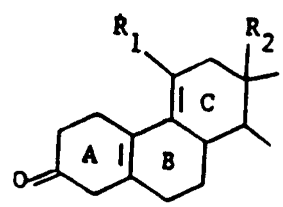

d) - or A and B represent the group:

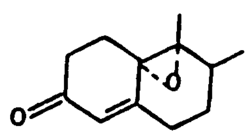

e) - or A and B represent the group:

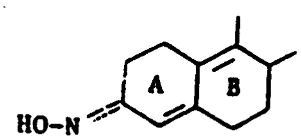

as well as their salts, characterized in that:
a) to prepare the products of formula ($I_A$):

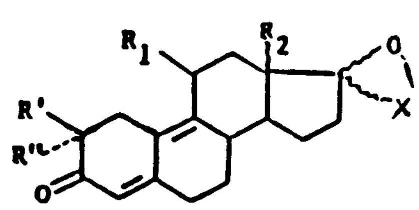

($I_A$)

in which $R_1$, X and $R_2$ keep the same meaning as in claim 1, R' and R" each represent a hydrogen atom or an alkyl radical or one represents a hydrogen atom and the other represents an alkyl radical, a product of formula (II):

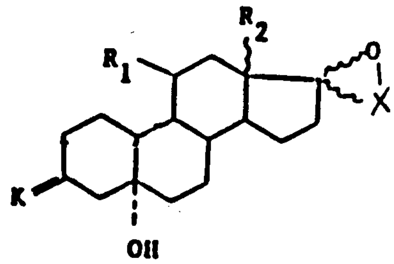

(II)

in which K represents a blocked ketone group, is subjected to the action of a dehydration reagent also capable of releasing the ketone function, in order to obtain the products of formula ($I_A$) in which R' and R" represent a hydrogen atom, and in that, if desired, the products of formula ($I_A$) in which $R_1$ contains a sulphur or nitrogen atom are subjected to an oxidation in order to obtain the products in which $R_1$ contains a sulphur atom oxidized into a sulphoxide or into a sulphone or a nitrogen atom oxidized into an N-oxide, and if desired, the products of formula ($I_A$) are subjected to the action of a strong base then of an alkyl halide in order to obtain a product of formula ($I_A$) in which R' and/or R" represent an alkyl radical having 1 to 4 carbon atoms;

b) to prepare the products of formula ($I_B$):

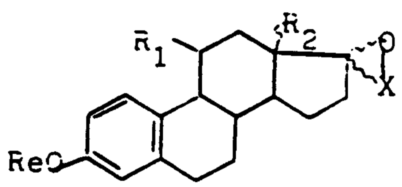

($I_B$)

in which $R_1$, $R_2$ and Re keep the same meaning as in claim 1, a product of formula ($I'_A$):

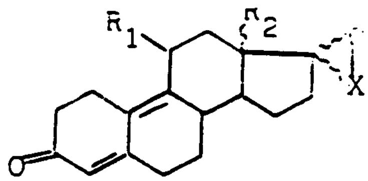

($I'_A$)

in which $R_1$ and $R_2$ have the meaning already indicated, obtained as described above, is subjected to the action of an aromatization agent then if appropriate to the action of a saponification agent and finally if desired the product of formula ($I_B$) in which Re is a hydrogen atom is subjected to an alkylation reagent;

c) to prepare the products of formula ($I_C$):

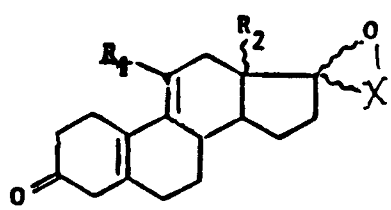

($I_C$)

in which $R_1$ and $R_2$ keep the same meaning as in claim 1, a product of formula ($I'_A$) is subjected to the action of an acylation agent then of a saponification agent;

d) to prepare the products of formula ($I_D$):

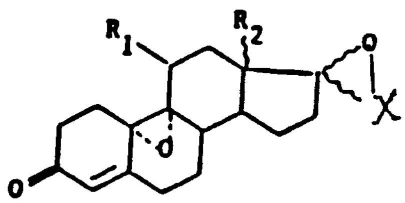

($I_D$)

in which $R_1$ and $R_2$ keep the same meaning as in claim 1, a product of formula ($I'_A$) is subjected to the action of an epoxidation agent;

e) to prepare the products of formula ($I_E$):

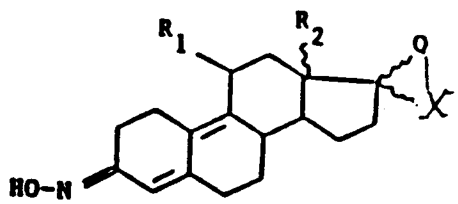

($I_E$)

in which $R_1$ and $R_2$ keep the same meaning as in claim 1, hydroxylamine is reacted on a product of formula ($I'_A$);

f) if appropriate, the products of formulae ($I_A$), ($I_B$), ($I_C$), ($I_D$) and ($I_E$) obtained are salified.

**2.** Preparation process for the products of general formula (I) as defined in claim 1, in which X represents a group:
$-(CH_2)_4-$ or $-CH=CH-CH_2-CH_2-$ characterized in that a product of formula (III):

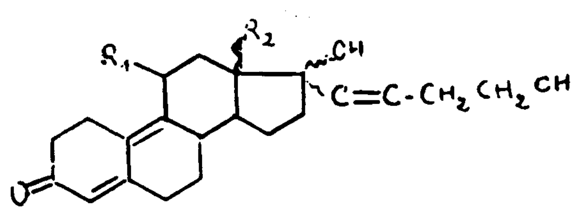

(III)

in which $R_1$ and $R_2$ keep the same meaning as in claim 1 and the wavy line indicates the optional presence of a double bond between the carbons which carry it, is subjected to the action of a cyclization reagent, in order to obtain a product of formula ($I''_A$):

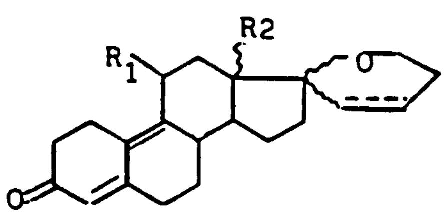

($I''_A$)

corresponding to a product of formula ($I_A$) in which R' and R" each represent a hydrogen atom and X represents the groups $-(CH_2)_4$ and $-CH=CH-CH_2-CH_2-$, which products of formula ($I''_A$) are converted into corresponding products of formula ($I_A$) in which one of the radicals R' or R" or both radicals R' and

R" represent an alkyl radical having 1 to 4 carbon atoms, and into products of formulae $I_B$, $I_C$, $I_D$ and $I_E$ according to the process described in claim 1.

**3.** Process according to claim 1, characterized in that a product of formula (II) is used at the start in which $R_1$ represents an optionally substituted carbocyclic or heterocyclic aryl or aralkyl radical, $R_2$ in the beta position represents a hydrocarbon radical containing 1 to 18 carbon atoms, the oxygen atom of the spiro ether is in the 17beta position, X represents a $-(CH_2)_n$ radical in which n represents an integer equal to 2 or 4 or X represents a $-CH=CH-CH_2-CH_2-$ radical.

**4.** Process according to claim 2, characterized in that a product of formula (III) is used at the start in which $R_1$ represents an optionally substituted carbocyclic or heterocyclic aryl or aralkyl radical, $R_2$ in the beta position represents a hydrocarbon radical containing 1 to 18 carbon atoms, the oxygen atom of the spiro ether is in the 17beta position.

**5.** Process according to any one of claims 1 to 4, characterized in that a product of formula (II) or (III) is used at the start in which $R_1$ represents either an aryl or aralkyl radical carrying an amine function:

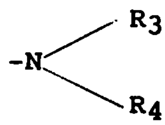

in which $R_3$ and $R_4$ each represent a primary, secondary or tertiary alkyl radical containing 1 to 8 carbon atoms or $R_3$ and $R_4$ form with the nitrogen atom to which they are linked a heterocycle optionally containing another heteroatom chosen from the group formed by oxygen, nitrogen, sulphur and silicon, or an aryl radical carrying a methylthio or ethylthio function.

**6.** Process according to any one of claims 1 to 5, characterized in that a product of formula (II) or (III) is used at the start in which $R_1$ is a phenyl radical, the substituent carried by this phenyl radical is in the para position.

**7.** Process according to any one of claims 1 to 6, characterized in that a product of formula (II) or (III) is used at the start in which $R_1$ represents one of the following radicals:

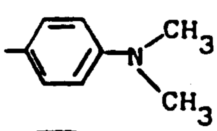 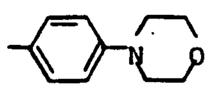 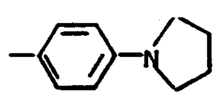 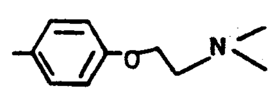

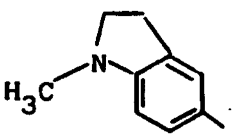 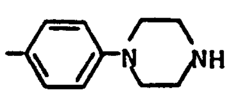 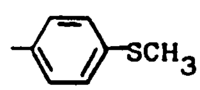 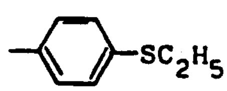

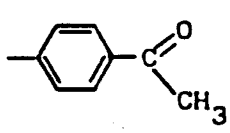 and 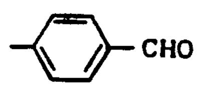

**8.** Process according to any one of claims 1 to 7, characterized in that a product of formula (II) or (III) is used at the start in which $R_1$ represents a radical:

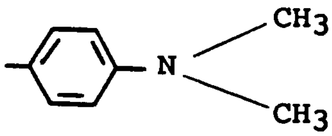

or a radical:

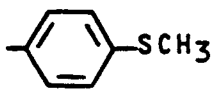

**9.** Process according to claim 8, characterized in that the product of formula (II) or (III) is chosen in such a way that the following are prepared:
- (17R) 11beta-[4-(dimethylamino)phenyl] spiro (estra-4,9-dien-17,2'-oxetan)-3-one,
- (17R) 11beta-[4-(methylthio)phenyl] spiro (estra-4,9-dien-17,2'-oxetan)-3-one.

**10.** Preparation process for pharmaceutical compositions, characterized in that at least one of the products of formula (I) as defined in claim 1, or at least one of their pharmaceutically acceptable salts, is used as active ingredient, in a form intended for this use.

**11.** Preparation process for pharmaceutical compositions, characterized in that at least one of the products of formula (I) as defined in any one of claims 3 to 8, or at least one of their pharmaceutically acceptable salts, is used as active ingredient in a form intended for this use.

**12.** Preparation process for pharmaceutical compositions, characterized in that at least one of the products of formula (I) as defined in claim 9, or at least one of their pharmaceutically acceptable salts, is used as active ingredient, in a form intended for this use.

**13.** As new industrial products, the products of formula (II'):

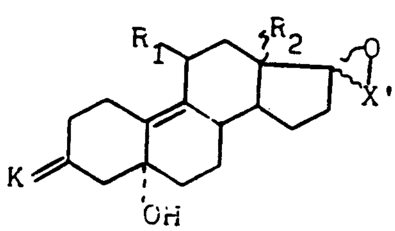

(II')

in which $R_1$, $R_2$ and K have the meaning indicated in claim 8 and X' represents $-(CH_2)_{n1}-$ in which n1 = 2 or 4 or X' represents: $-CH=CH-CH_2-CH_2$.

**Patentansprüche**

**Patentansprüche für folgende Vertragsstaaten : AT, BE, CH, DE, FR, GB, IT, LI, LU, NL, SE**

1. Produkte der allgemeinen Formel (I)

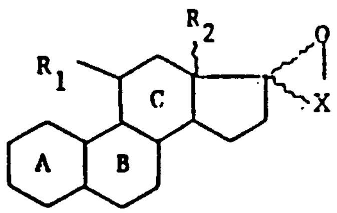

(I)

worin $R_1$ für einen gegebenenfalls substituierten carbocyclischen oder heterocyclischen Aryl- oder Aralkylrest steht, $R_2$ in Alpha- oder Betastellung einen Kohlenwasserstoffrest mit 1 bis 18 Kohlenstoffatomen wiedergibt, die gewellte Linie des Spiroethers anzeigt, daß sich das Sauerstoffatom in Alpha- oder Betastellung befinden kann, X für einen Rest $-(CH_2)_n-$ steht, worin n eine ganze Zahl entsprechend 1, 2 oder 4 bedeutet, oder X einen Rest $-CH=CH-CH_2-CH_2-$ wiedergibt, wobei die Ringe A und B eine der folgenden Strukturen besitzen:

a) - entweder stellen A und B die Gruppe

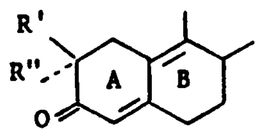

dar, worin R' und R", die identisch oder voneinander verschieden sind, ein Wasserstoffatom oder einen Alkylrest mit 1 bis 4 Kohlenstoffatomen bedeuten,

b) - oder A und B stellen die Gruppe

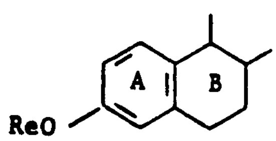

dar, worin Re für ein Wasserstoffatom, einen gegebenenfalls substituierten Alkylrest mit 1 bis 6 Kohlenstoffatomen oder einen Acylrest steht;

c) - oder A, B und C stellen die Gruppe

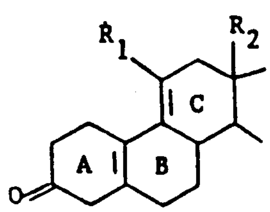

dar,

d) - oder A und B stellen die Gruppe

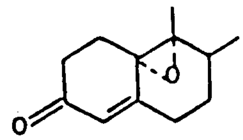

dar,

e) - oder A und B stellen die Gruppe

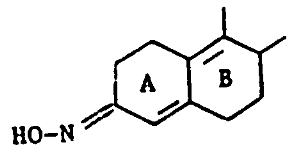

dar, sowie deren Salze.

**2.** Produkte der Formel (I), wie in Anspruch 1 definiert, mit der Formel (I')

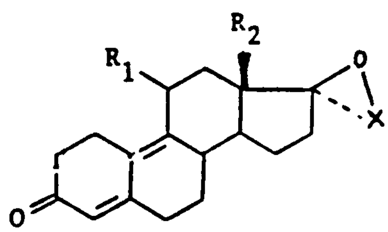

(I').

worin $R_1$ für einen gegebenenfalls substituierten carbocyclischen oder heterocyclischen Aryl- oder Aralkylrest steht, $R_2$ in Betastellung einen Kohlenwasserstoffrest mit 1 bis 18 Kohlenstoffatomen bedeutet, das Sauerstoffatom des Spiroethers sich in 17-Betastellung befindet, X für einen Rest $-(CH_2)_n-$steht, worin n eine ganze Zahl entsprechend 2 oder 4 bedeutet, oder X einen Rest $-CH=CH-CH_2-CH_2-$ wiedergibt, sowie deren Salze.

**3.** Produkte der Formel (I), wie in einem der Ansprüche 1 oder 2 definiert, worin $R_1$ entweder einen Aryl- oder Aralkylrest bedeutet mit einer Aminfunktion

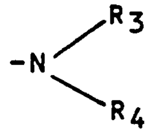

worin $R_3$ und $R_4$ jeweils einen primären, sekundären oder tertiären Alkylrest mit 1 bis 8 Kohlenstoffatomen bedeuten, oder $R_3$ und $R_4$ mit dem Stickstoffatom, an das sie gebunden sind, einen Heterocyclus bilden, der gegebenenfalls ein weiteres Heteroatom, ausgewählt unter Sauerstoff, Stickstoff, Schwefel und Silicium, enthält, oder einen Arylrest mit einer Methylthio- oder Ethylthiofunktion wiedergibt, sowie deren Salze.

**4.** Produkte der Formel (I), wie in einem der Ansprüche 1 bis 3 definiert, worin $R_1$ einen Phenylrest bedeutet, und der von diesem Phenylrest getragene Substituent sich in para-Stellung befindet, sowie

deren Salze.

**5.** Produkte der Formel (I), wie in einem der Ansprüche 1 bis 4 definiert, worin $R_1$ einen der folgenden Reste wiedergibt

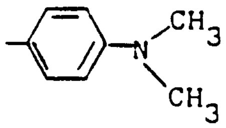 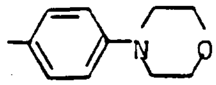 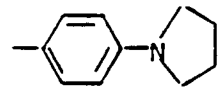 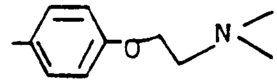

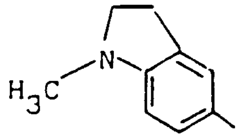 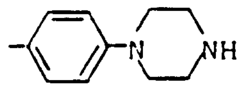 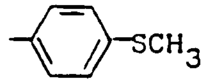 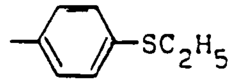

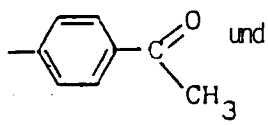 und 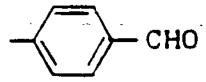

sowie deren Salze.

**6.** Produkte der Formel (I), wie in Anspruch 5 definiert, worin $R_1$ einen Rest

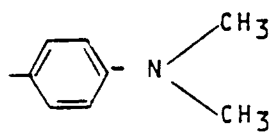

oder einen Rest

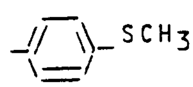

wiedergibt, sowie deren Salze.

**7.** Produkte der Formel (I), wie in einem der Ansprüche 1 bis 6 definiert, mit den folgenden Bezeichnungen:

- (17R),11β-[4-(Dimethylamino)-phenyl]-spiro-(östra-4,9-dien-17,2'-oxetan)-3-on,
- (17R),11β-[4-(Methylthio)-phenyl]-spiro-(östra-4,9-dien-17,2'-oxetan)-3-on.

**8.** Verfahren zur Herstellung der Produkte der allgemeinen Formel (I), wie in Anspruch 1 definiert, dadurch gekennzeichnet, daß man

a) zur Herstellung der Produkte der Formel ($I_A$)

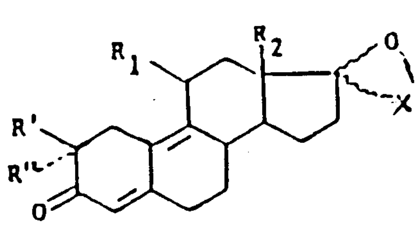 ($I_A$).

worin $R_1$, X und $R_2$ die in Anspruch 1 angegebene Bedeutung besitzen, R' und R" jeweils ein Wasserstoffatom oder einen Alkylrest bedeuten, oder aber eines ein Wasserstoffatom und das andere einen Alkylrest bedeutet, ein Produkt der Formel (II)

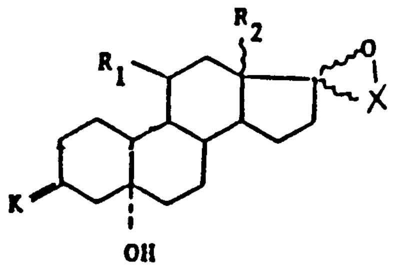

(II)

worin K eine blockierte Ketogruppe wiedergibt, der Einwirkung eines Dehydratationsmittels unterzieht, das auch in der Lage ist, die Ketofunktion freizusetzen, um zu den Produkten der Formel ($I_A$) zu gelangen, worin R' und R" ein Wasserstoffatom bedeuten, und gewünschtenfalls die Produkte der Formel ($I_A$),worin $R_1$ ein Schwefel- oder Stickstoffatom enthält, einer Oxidation unterzieht, um die Produkte zu erhalten, worin $R_1$ ein zum Sulfoxid oder zum Sulfon oxidiertes Schwefelatom oder ein zum N-oxid oxidiertes Stickstoffatom enthält, und gewünschtenfalls die Produkte der Formel ($I_A$) der Einwirkung einer starken Base, danach derjenigen eines Alkylhalogenids unterzieht, um zu einem Produkt der Formel ($I_A$) zu gelangen, worin R' und /oder R" einen Alkylrest mit 1 bis 4 Kohlenstoffatomen bedeuten;

b) zur Herstellung der Produkte der Formel ($I_B$)

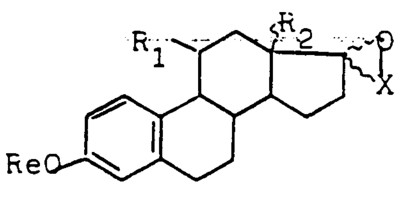

($I_B$)

worin $R_1$, $R_2$ und Re die in Anspruch 1 angegebene Bedeutung besitzen, ein Produkt der Formel ($I'_A$)

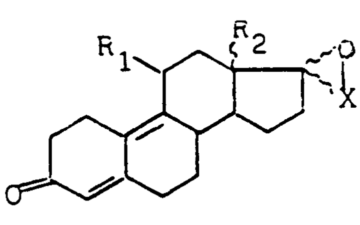

($I'_A$)

worin $R_1$ und $R_2$ die angegebene Bedeutung besitzen, und das wie vorstehend erhalten wurde, der Einwirkung eines Aromatisierungsmittels, danach gegebenenfalls der Einwirkung eines Verseifungsmittels, unterwirft, und schließlich gewünschtenfalls das Produkt der Formel ($I_B$), worin Re für ein Wasserstoffatom steht, einem Alkylierungsreagens unterzieht;

c) zur Herstellung der Produkte der Formel ($I_C$)

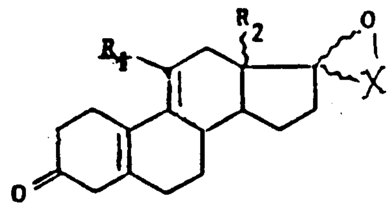

($I_C$)

worin $R_1$ und $R_2$ die in Anspruch 1 angegebene Bedeutung besitzen, ein Produkt der Formel ($I'_A$) der Einwirkung eines Acylierungsmittels, danach eines Verseifungsmittels, unterzieht;

d) zur Herstellung der Produkte der Formel ($I_D$)

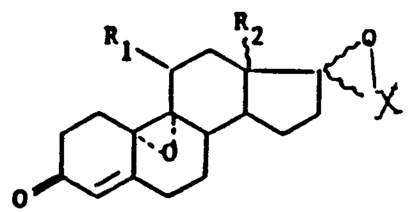

($I_D$)

worin $R_1$ und $R_2$ die in Anspruch 1 angegebene Bedeutung besitzen, ein Produkt der Formel ($I'_A$) der Einwirkung eines Epoxidierungsmittels unterzieht;

e) zur Herstellung der Produkte der Formel ($I_E$)

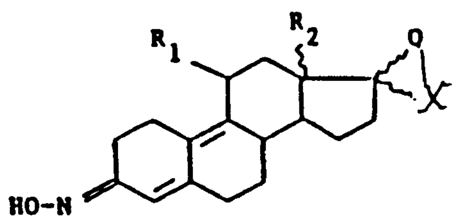

($I_E$)

worin $R_1$ und $R_2$ die in Anspruch 1 angegebene Bedeutung besitzen, ein Hydroxylamin mit einem Produkt der Formel ($I'_A$) umsetzt;

f) gegebenenfalls die erhaltenen Produkte der Formel ($I_A$), ($I_B$), ($I_C$), ($I_D$) und ($I_E$) in ein Salz überführt.

**9.** Verfahren zur Herstellung der Produkte der allgemeinen Formel (I), wie in Anspruch 1 definiert, worin X für eine Gruppe $-(CH_2)_4-$ oder $-CH=CH-CH_2-CH_2-$ steht, dadurch gekennzeichnet, daß man ein Produkt der Formel (III)

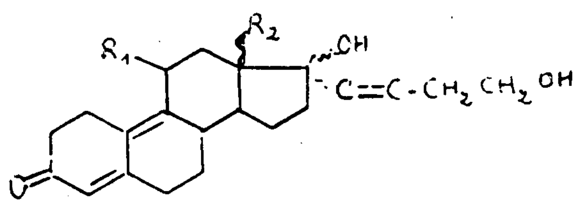

(III)

worin $R_1$ und $R_2$ die in Anspruch 1 angegebene Bedeutung besitzen, und die gestrichelte Linie die etwaige Anwesenheit einer Doppelbindung zwischen den Kohlenstoffatomen, die sie tragen, anzeigt, der Einwirkung eines Cyclisierungsmittels unterzieht, um ein Produkt der Formel ($I''_A$)

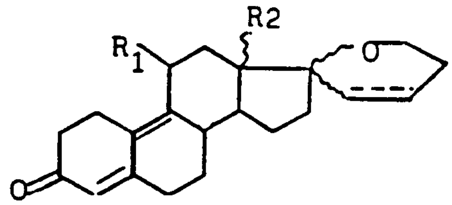

(I"$_A$)

entsprechend einem Produkt der Formel ($I_A$), worin R' und R" jeweils ein Wasserstoffatom bedeuten, und X die Gruppen $-(CH_2)_4-$ und $-CH=CH-CH_2-CH_2-$ wiedergibt, zu erhalten, die Produkte der Formel (I"$_A$) in die entsprechenden Produkte der Formel ($I_A$), worin eines der Reste R' oder R" oder beide Reste R' und R" einen Alkylrest mit 1 bis 4 Kohlenstoffatomen bedeuten, und in die Produkte der Formeln ($I_B$), ($I_C$) ($I_D$) und ($I_E$) entsprechend dem in Anspruch 8 beschriebenen Verfahren überführt.

**10.** Als Arzneimittel die Produkte der allgemeinen Formel (I), wie in Anspruch 1 definiert, sowie deren pharmazeutisch verträgliche Salze.

**11.** Als Arzneimittel die Produkte der allgemeinen Formel (I), wie in einem der Ansprüche 2 bis 6 definiert, sowie deren pharmazeutisch verträgliche Salze.

**12.** Als Arzneimittel die Produkte der allgemeinen Formel (I), wie in Anspruch 7 definiert, sowie deren pharmazeutisch verträgliche Salze.

**13.** Pharmazeutische Zusammensetzungen, enthaltend als Wirkstoff zumindest eines der Arzneimittel gemäß einem der Ansprüche 10 bis 12.

**14.** Als neue industrielle Produkte die Produkte der Formel (II')

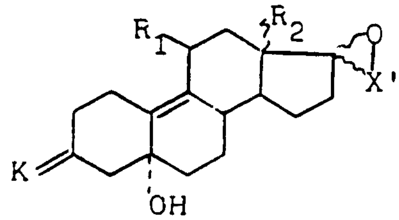

(II')

worin $R_1$, $R_2$ und K die in Anspruch 8 angegebene Bedeutung besitzen, und X' für $-(CH_2)_{n1}-$ steht, worin $n_1$ = 2 oder 4, oder X' für $-CH=CH-CH_2-CH_2-$ steht.

**Patentansprüche für folgenden Vertragsstaat : ES**

**1.** Verfahren zur Herstellung der Produkte der allgemeinen Formel (I)

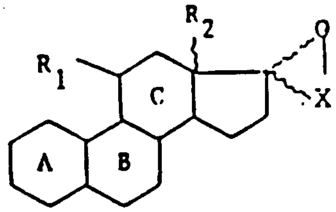

(I)

worin $R_1$ einen gegebenenfalls substituierten carbocyclischen oder heterocyclischen Aryl- oder Aralkyl-

rest bedeutet, $R_2$ in Alpha- oder Betastellung einen Kohlenwasserstoffrest mit 1 bis 18 Kohlenstoffatomen wiedergibt, die gewellte Linie des Spiroethers anzeigt, daß sich das Sauerstoffatom in Alpha- oder Betastellung befinden kann, X für einen Rest $-(CH_2)_n-$ steht, worin n eine ganze Zahl entsprechend 1, 2 oder 4 bedeutet, oder X einen Rest $-CH=CH-CH_2-CH_2-$ bedeutet, wobei die Ringe A und B eine der folgenden Strukturen aufweisen:

a) - entweder bedeuten A und B die Gruppe

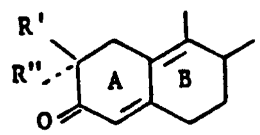

worin R' und R", die identisch oder voneinander verschieden sind, für ein Wasserstoffatom oder einen Alkylrest mit 1 bis 4 Kohlenstoffatomen stehen,

b) - oder A und B bedeuten die Gruppe

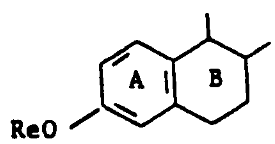

worin Re für ein Wasserstoffatom, einen gegebenenfalls substituierten Alkylrest mit 1 bis 6 Kohlenstoffatomen oder einen Acylrest steht;

c) - oder A, B und C bedeuten die Gruppe

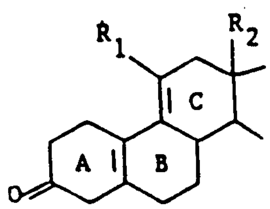

d) - oder A und B bedeuten die Gruppe

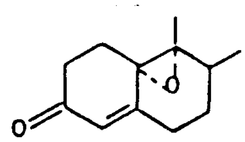

e) - oder A und B bedeuten die Gruppe

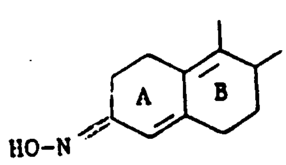

sowie von deren Salzen, dadurch gekennzeichnet, daß man:

a) zur Herstellung der Produkte der Formel ($I_A$)

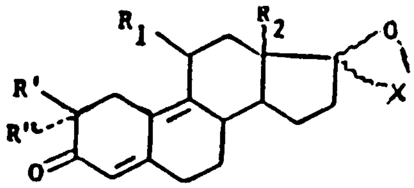

($I_A$)

worin $R_1$ und $R_2$ die vorstehend angegebene Bedeutung besitzen, R' und R" jeweils ein Wasserstoffatom oder einen Alkylrest bedeuten, oder aber eines ein Wasserstoffatom und das andere einen Alkylrest bedeutet, ein Produkt der Formel (II)

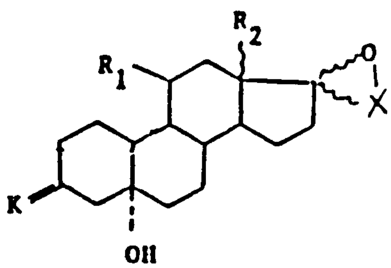

(II)

worin K für eine blockierte Ketogruppe steht, der Einwirkung eines Dehydratationsmittels unterzieht, das auch in der Lage ist, die Ketofunktion freizusetzen, um zu den Produkten der Formel ($I_A$) zu gelangen, worin R' und R" für ein Wasserstoffatom stehen, und gewünschtenfalls die Produkte der Formel ($I_A$), worin $R_1$ ein Schwefel- oder Stickstoffatom bedeutet, einer Oxidation unterzieht, um zu den Produkten zu gelangen, worin $R_1$ ein zu einem Sulfoxid oder einem Sulfon oxidiertes Schwefelatom oder ein zu einem N-oxid oxidiertes Stickstoffatom enthält, und gewünschtenfalls die Produkte der Formel ($I_A$) der Einwirkung einer starken Base, danach derjenigen eines Alkylhalogenids, unterzieht, um zu einem Produkt der Formel ($I_A$) zu gelangen, worin R' und/oder R" für einen Alkylrest mit 1 bis 4 Kohlenstoffatomen stehen;

b) zur Herstellung der Produkte der Formel ($I_B$)

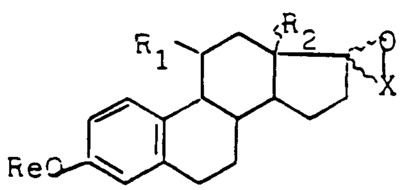

($I_B$)

worin $R_1$, $R_2$ und Re die vorstehend angegebene Bedeutung besitzen, ein Produkt der Formel ($I'_A$)

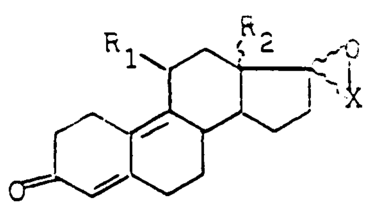

($I'_A$)

worin $R_1$ und $R_2$ die angegebene Bedeutung besitzen, und das wie vorstehend beschrieben

erhalten wurde, der Einwirkung eines Aromatisierungsmittels, danach gegebenenfalls der Einwirkung eines Verseifungsmittels, unterzieht, und schließlich gewünschtenfalls das Produkt der Formel ($I_B$), worin Re ein Wasserstoffatom bedeutet, einem Alkylierungsreagens unterzieht;
c) zur Herstellung der Produkte der Formel ($I_C$)

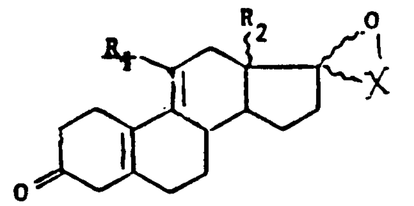

($I_C$)

worin $R_1$ und $R_2$ die vorstehend angegebene Bedeutung besitzen, ein Produkt der Formel ($I'_A$) der Einwirkung eines Acylierungsmittels, danach derjenigen eines Verseifungsmittels, unterzieht;
d) zur Herstellung der Produkte der Formel ($I_D$)

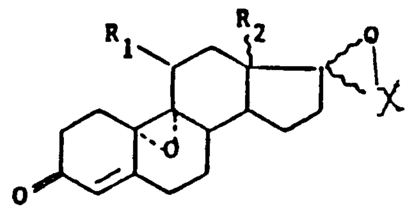

($I_D$)

worin $R_1$ und $R_2$ die vorstehend angegebene Bedeutung besitzen, ein Produkt der Formel ($I'_A$) der Einwirkung eines Epoxidierungsmittels unterzieht;
e) zur Herstellung der Produkte der Formel ($I_E$)

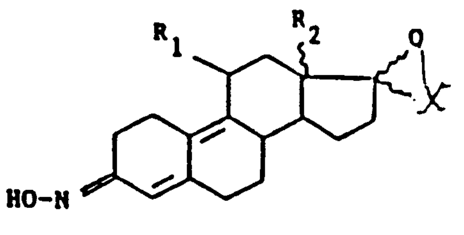

($I_E$)

worin $R_1$ und $R_2$ die vorstehend angegebene Bedeutung besitzen, Hydroxylamin mit einem Produkt der Formel ($I'_A$) umsetzt;
f) gegebenenfalls die erhaltenen Produkte der Formel ($I_A$), ($I_B$), ($I_C$), ($I_D$) und ($I_E$) verseift.

**2.** Verfahren zur Herstellung der Produkte der allgemeinen Formel (I), wie in Anspruch 1 definiert, worin X eine Gruppe $-(CH_2)_4-$ oder $-CH=CH-CH_2-CH_2-$ bedeutet, dadurch gekennzeichnet, daß man ein Produkt der Formel (III)

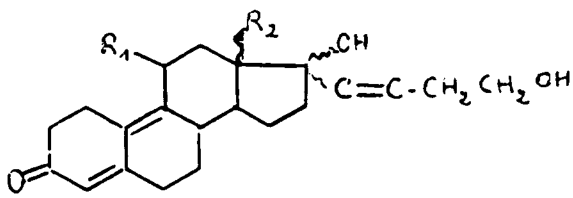

(III)

worin $R_1$ und $R_2$ die in Anspruch 1 angegebene Bedeutung besitzen, und die gestrichelte Linie die etwaige Anwesenheit einer Doppelbindung zwischen den Kohlenstoffatomen, die sie tragen, anzeigt, der Einwirkung eines Cyclisierungsmittels unterzieht, um zu einem Produkt der Formel ($I''_A$)

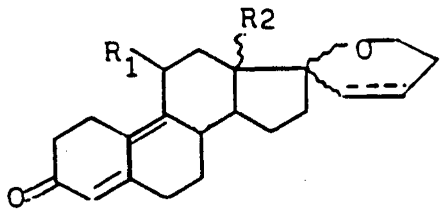

($I''_A$)

entsprechend einem Produkt der Formel ($I_A$), zu gelangen, worin R' und R" jeweils ein Wasserstoffatom bedeuten, und X für die Gruppen $-(CH_2)_4-$ und $-CH=CH-CH_2-CH_2-$ steht, die Produkte der Formel ($I''_A$) in die entsprechenden Produkte der Formel ($I_A$), worin einer der Reste R' oder R" oder beide Reste R' und R" einen Alkylrest mit 1 bis 4 Kohlenstoffatomen bedeuten, und in die Produkte der Formeln ($I_B$), ($I_C$), ($I_D$) und ($I_E$) gemäß dem in Anspruch 1 beschriebenen Verfahren überführt.

**3.** Verfahren gemäß Anspruch 1, dadurch gekennzeichnet, daß man von einem Produkt der Formel (II) ausgeht, worin $R_1$ einen gegebenenfalls substituierten carbocyclischen oder heterocyclischen Aryl- oder Aralkylrest bedeutet, $R_2$ in Betastellung für einen Kohlenwasserstoffrest mit 1 bis 18 Kohlenstoffatomen steht, das Sauerstoffatom des Spiroethers sich in 17-Betastellung befindet, X für einen Rest $-(CH_2)_n-$ steht, worin n eine ganze Zahl entsprechend 2 oder 4 bedeutet, oder X einen Rest $-CH=CH-CH_2-CH_2-$bedeutet.

**4.** Verfahren gemäß Anspruch 2, dadurch gekennzeichnet, daß man von einem Produkt der Formel (III) ausgeht, worin $R_1$ einen gegebenenfalls substituierten carbocyclischen oder heterocyclischen Aryl- oder Aralkylrest bedeutet, $R_2$ in Betastellung für einen Kohlenwasserstoffrest mit 1 bis 18 Kohlenstoffatomen steht, und das Sauerstoffatom des Spiroethers sich in 17-Betastellung befindet.

**5.** Verfahren gemäß einem der Ansprüche 1 bis 4, dadurch gekennzeichnet, daß man von einem Produkt der Formel (II) oder (III) ausgeht, worin $R_1$ entweder einen Aryl- oder Aralkylrest mit einer Aminfunktion

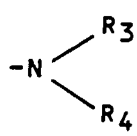

worin $R_3$ und $R_4$ jeweils einen primären, sekundären oder tertiären Alkylrest mit 1 bis 8 Kohlenstoffatomen bedeuten, oder $R_3$ und $R_4$ mit dem Stickstoffatom, an das sie gebunden sind, einen Heterocyclus bilden, der gegebenenfalls ein weiteres Heteroatom, ausgewählt unter Sauerstoff, Stickstoff, Schwefel und Silicium, enthält, oder einen Arylrest mit einer Methylthio- oder Ethylthiofunktion bedeutet.

**6.** Verfahren gemäß einem der Ansprüche 1 bis 5, dadurch gekennzeichnet, daß man von einem Produkt der Formel (II) oder (III) ausgeht, worin $R_1$ einen Phenylrest bedeutet, und der von diesem Phenylrest getragene Substituent sich in para-Stellung befindet.

7. Verfahren gemäß einem der Ansprüche 1 bis 6, dadurch gekennzeichnet, daß man von einem Produkt der Formel (II) oder (III) ausgeht, worin $R_1$ einen der folgenden Reste bedeutet

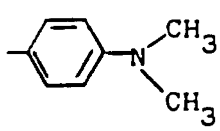
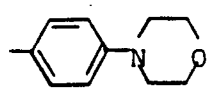
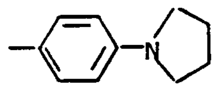
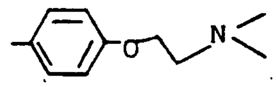

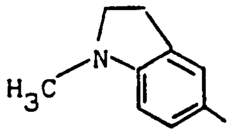
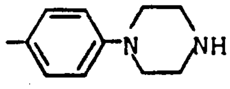
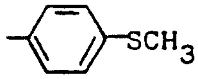
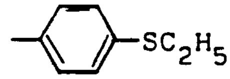

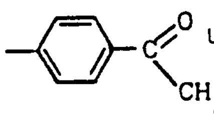 und 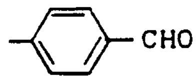

8. Verfahren gemäß einem der Ansprüche 1 bis 7, dadurch gekennzeichnet, daß man von einem Produkt der Formel (II) oder (III) ausgeht, worin $R_1$ einen Rest

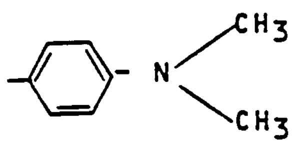

oder einen Rest

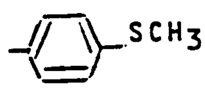

bedeutet.

9. Verfahren gemäß Anspruch 8, dadurch gekennzeichnet, daß man das Produkt der Formel (II) oder (III) derart auswählt, daß man herstellt:
(17R),11β-[4-(Dimethylamino)-phenyl]-spiro-(östra-4,9-dien-17,2'-oxetan)-3-on,
(17R),11β-[4-(Methylthio)-phenyl]-spiro-(östra-4,9-dien-17,2'-oxetan)-3-on.

**Patentansprüche für folgenden Vertragsstaat : GR**

1. Verfahren zur Herstellung der Produkte der allgemeinen Formel (I)

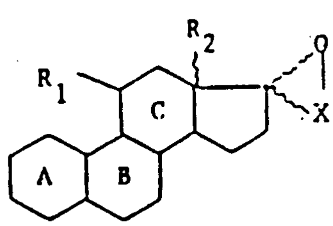

(I)

worin $R_1$ einen gegebenenfalls substituierten carbocyclischen oder heterocyclischen Aryl- oder Aralkylrest bedeutet, $R_2$ in Alpha- oder Betastellung einen Kohlenwasserstoffrest mit 1 bis 18 Kohlenstoffatomen wiedergibt, die gewellte Linie des Spiroethers anzeigt, daß sich das Sauerstoffatom in Alpha- oder Betastellung befinden kann, X für einen Rest $-(CH_2)_n-$ steht, worin n eine ganze Zahl entsprechend 1, 2 oder 4 bedeutet, oder X einen Rest $-CH=CH-CH_2-CH_2-$ bedeutet, wobei die Ringe A und B eine der folgenden Strukturen aufweisen:

a) - entweder bedeuten A und B die Gruppe

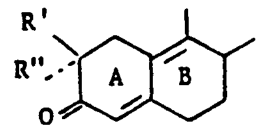

worin R' und R", die identisch oder voneinander verschieden sind, für ein Wasserstoffatom oder einen Alkylrest mit 1 bis 4 Kohlenstoffatomen stehen,

b) - oder A und B bedeuten die Gruppe

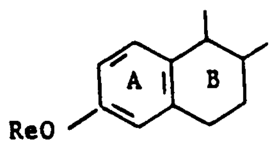

worin Re für ein Wasserstoffatom, einen gegebenenfalls substituierten Alkylrest mit 1 bis 6 Kohlenstoffatomen oder einen Acylrest steht;

c) - oder A, B und C bedeuten die Gruppe

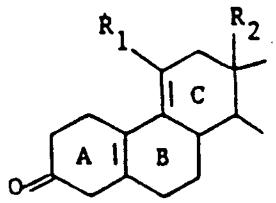

d) - oder A und B bedeuten die Gruppe

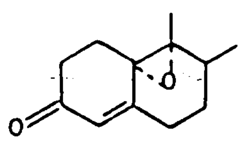

e) - oder A und B bedeuten die Gruppe

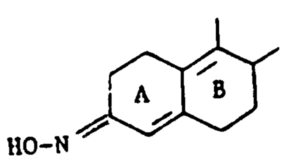

sowie von deren Salzen, dadurch gekennzeichnet, daß man:

a) zur Herstellung der Produkte der Formel ($I_A$)

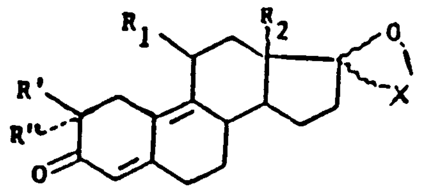

($I_A$)

worin $R_1$ und $R_2$ die vorstehend angegebene Bedeutung besitzen, R' und R" jeweils ein Wasserstoffatom oder einen Alkylrest bedeuten, oder aber eines ein Wasserstoffatom und das andere einen Alkylrest bedeutet, ein Produkt der Formel (II)

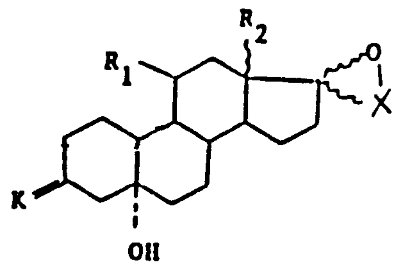

(II)

worin K für eine blockierte Ketogruppe steht, der Einwirkung eines Dehydratationsmittels unterzieht, das auch in der Lage ist, die Ketofunktion freizusetzen, um zu den Produkten der Formel ($I_A$) zu gelangen, worin R' und R" für ein Wasserstoffatom stehen, und gewünschtenfalls die Produkte der Formel ($I_A$), worin $R_1$ ein Schwefel- oder Stickstoffatom bedeutet, einer Oxidation unterzieht, um zu den Produkten zu gelangen, worin $R_1$ ein zu einem Sulfoxid oder einem Sulfon oxidiertes Schwefelatom oder ein zu einem N-oxid oxidiertes Stickstoffatom enthält, und gewünschtenfalls die Produkte der Formel ($I_A$) der Einwirkung einer starken Base, danach derjenigen eines Alkylhalogenids, unterzieht, um zu einem Produkt der Formel ($I_A$) zu gelangen, worin R' und/oder R" für einen Alkylrest mit 1 bis 4 Kohlenstoffatomen stehen;

b) zur Herstellung der Produkte der Formel ($I_B$)

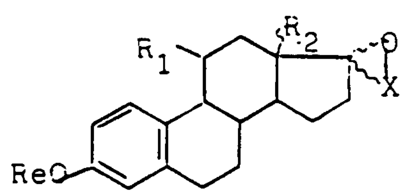

($I_B$)

worin $R_1$, $R_2$ und Re die vorstehend angegebene Bedeutung besitzen, ein Produkt der Formel ($I'_A$)

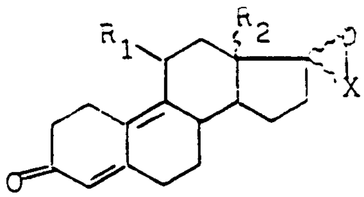

(I'$_A$)

worin $R_1$ und $R_2$ die angegebene Bedeutung besitzen, und das wie vorstehend beschrieben erhalten wurde, der Einwirkung eines Aromatisierungsmittels, danach gegebenenfalls der Einwirkung eines Verseifungsmittels, unterzieht, und schließlich gewünschtenfalls das Produkt der Formel ($I_B$), worin Re ein Wasserstoffatom bedeutet, einem Alkylierungsreagens unterzieht;
c) zur Herstellung der Produkte der Formel ($I_C$)

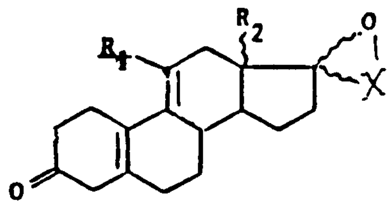

($I_C$)

worin $R_1$ und $R_2$ die vorstehend angegebene Bedeutung besitzen, ein Produkt der Formel (I'$_A$) der Einwirkung eines Acylierungsmittels, danach derjenigen eines Verseifungsmittels, unterzieht;
d) zur Herstellung der Produkte der Formel ($I_D$)

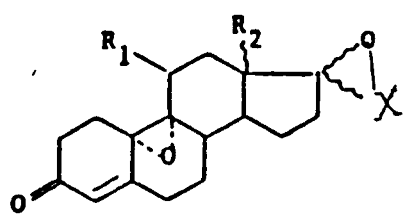

($I_D$)

worin $R_1$ und $R_2$ die vorstehend angegebene Bedeutung besitzen, ein Produkt der Formel (I'$_A$) der Einwirkung eines Epoxidierungsmittels unterzieht;
e) zur Herstellung der Produkte der Formel ($I_E$)

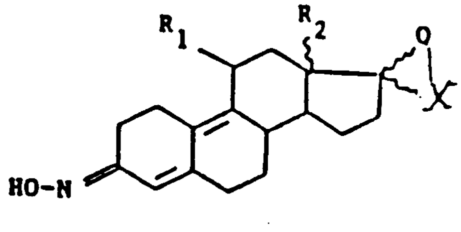

($I_E$)

worin $R_1$ und $R_2$ die vorstehend angegebene Bedeutung besitzen, Hydroxylamin mit einem Produkt der Formel (I'$_A$) umsetzt;
f) gegebenenfalls die erhaltenen Produkte der Formel ($I_A$), ($I_B$), ($I_C$), ($I_D$) und ($I_E$) verseift.

**2.** Verfahren zur Herstellung der Produkte der allgemeinen Formel (I), wie in Anspruch 1 definiert, worin X eine Gruppe $-(CH_2)_4-$ oder $-CH=CH-CH_2-CH_2-$ bedeutet, dadurch gekennzeichnet, daß man ein Produkt der Formel (III)

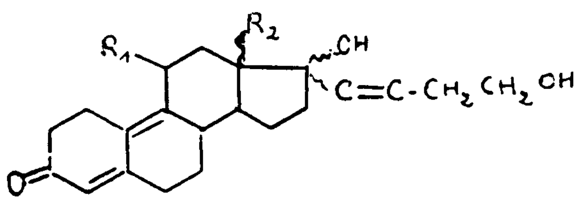

(III)

worin $R_1$ und $R_2$ die in Anspruch 1 angegebene Bedeutung besitzen, und die gestrichelte Linie die etwaige Anwesenheit einer Doppelbindung zwischen den Kohlenstoffatomen, die sie tragen, anzeigt, der Einwirkung eines Cyclisierungsmittels unterzieht, um zu einem Produkt der Formel ($I''_A$)

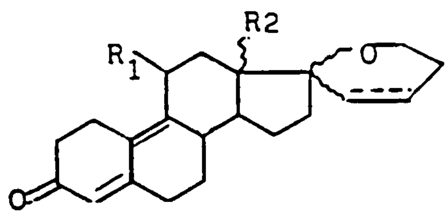

($I''_A$)

entsprechend einem Produkt der Formel ($I_A$), zu gelangen, worin R' und R" jeweils ein Wasserstoffatom bedeuten, und X für die Gruppen $-(CH_2)_4-$ und $-CH=CH-CH_2-CH_2-$ steht, die Produkte der Formel ($I''_A$) in die entsprechenden Produkte der Formel ($I_A$), worin einer der Reste R' oder R" oder beide Reste R' und R" einen Alkylrest mit 1 bis 4 Kohlenstoffatomen bedeuten, und in die Produkte der Formeln ($I_B$), ($I_C$), ($I_D$) und ($I_E$) gemäß dem in Anspruch 1 beschriebenen Verfahren überführt.

**3.** Verfahren gemäß Anspruch 1, dadurch gekennzeichnet, daß man von einem Produkt der Formel (II) ausgeht, worin $R_1$ einen gegebenenfalls substituierten carbocyclischen oder heterocyclischen Aryl- oder Aralkylrest bedeutet, $R_2$ in Betastellung für einen Kohlenwasserstoffrest mit 1 bis 18 Kohlenstoffatomen steht, das Sauerstoffatom des Spiroethers sich in 17-Betastellung befindet, X für einen Rest $-(CH_2)_n$-steht, worin n eine ganze Zahl entsprechend 2 oder 4 bedeutet, oder X einen Rest $-CH=CH-CH_2-CH_2-$ bedeutet.

**4.** Verfahren gemäß Anspruch 2, dadurch gekennzeichnet, daß man von einem Produkt der Formel (III) ausgeht, worin $R_1$ einen gegebenenfalls substituierten carbocyclischen oder heterocyclischen Aryl- oder Aralkylrest bedeutet, $R_2$ in Betastellung für einen Kohlenwasserstoffrest mit 1 bis 18 Kohlenstoffatomen steht, und das Sauerstoffatom des Spiroethers sich in 17-Betastellung befindet.

**5.** Verfahren gemäß einem der Ansprüche 1 bis 4, dadurch gekennzeichnet, daß man von einem Produkt der Formel (II) oder (III) ausgeht, worin $R_1$ entweder einen Aryl- oder Aralkylrest mit einer Aminfunktion

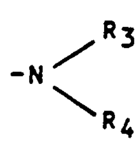

worin $R_3$ und $R_4$ jeweils einen primären, sekundären oder tertiären Alkylrest mit 1 bis 8 Kohlenstoffatomen bedeuten, oder $R_3$ und $R_4$ mit dem Stickstoffatom, an das sie gebunden sind, einen Heterocyclus

bilden, der gegebenenfalls ein weiteres Heteroatom, ausgewählt unter Sauerstoff, Stickstoff, Schwefel und Silicium, enthält, oder einen Arylrest mit einer Methylthio- oder Ethylthiofunktion bedeutet.

6. Verfahren gemäß einem der Ansprüche 1 bis 5, dadurch gekennzeichnet, daß man von einem Produkt der Formel (II) oder (III) ausgeht, worin $R_1$ einen Phenylrest bedeutet, und der von diesem Phenylrest getragene Substituent sich in para-Stellung befindet.

7. Verfahren gemäß einem der Ansprüche 1 bis 6, dadurch gekennzeichnet, daß man von einem Produkt der Formel (II) oder (III) ausgeht, worin $R_1$ einen der folgenden Reste bedeutet

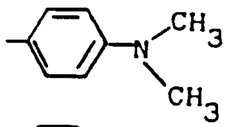 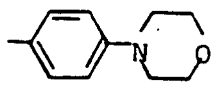 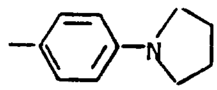 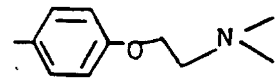

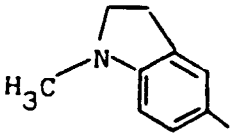 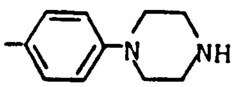 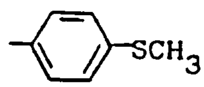 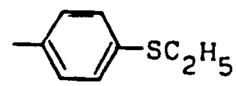

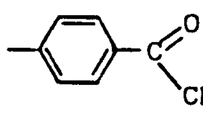 und 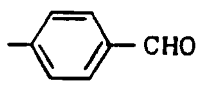

8. Verfahren gemäß einem der Ansprüche 1 bis 7, dadurch gekennzeichnet, daß man von einem Produkt der Formel (II) oder (III) ausgeht, worin $R_1$ einen Rest

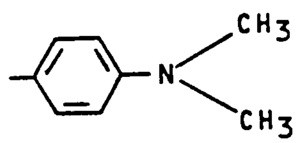

oder einen Rest

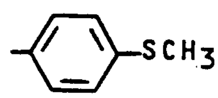

bedeutet.

9. Verfahren gemäß Anspruch 8, dadurch gekennzeichnet, daß man das Produkt der Formel (II) oder (III) derart auswählt, daß man herstellt:
(17R),11β-[4-(Dimethylamino)-phenyl]-spiro-(östra-4,9-dien-17,2'-oxetan)-3-on,
(17R),11β-[4-(Methylthio)-phenyl]-spiro-(östra-4,9-dien-17,2'-oxetan)-3-on.

10. Verfahren zur Herstellung von pharmazeutischen Zusammensetzungen, dadurch gekennzeichnet, daß man als Wirkstoff zumindest eines der Produkte der Formel (I), wie in Anspruch 1 definiert, oder zumindest eines ihrer pharmazeutisch verträglichen Salze in eine für diese Verwendung bestimmte Form überführt.

11. Verfahren zur Herstellung von pharmazeutischen Zusammensetzungen, dadurch gekennzeichnet, daß man als Wirkstoff zumindest eines der Produkte der Formel (I), wie in einem der Ansprüche 3 bis 8

definiert, oder zumindest eines ihrer pharmazeutisch verträglichen Salze in eine für diese Verwendung bestimmte Form überführt.

**12.** Verfahren zur Herstellung von pharmazeutischen Zusammensetzungen, dadurch gekennzeichnet, daß man als Wirkstoff zumindest eines der Produkte der Formel (I), wie in Anspruch 9 definiert, oder zumindest eines ihrer pharmazeutisch vertraglichen Salze in eine für diese Verwendung bestimmte Form überführt.

**13.** Als neue industrielle Produkte die Produkte der Formel (II')

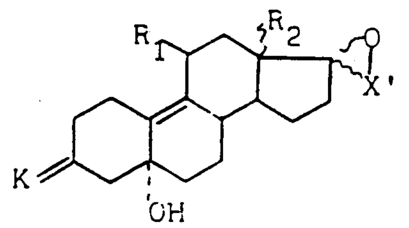

(II')

worin $R_1$, $R_2$ und K die in Anspruch 8 angegebene Bedeutung besitzen, und X' für $-(CH_2)_{n1}-$, worin $n_1$ = 2 oder 4, steht, oder X' für $-CH=CH-CH_2-CH_2-$ steht.